(19) Europäisches Patentamt
European Patent Office
Office européen des brevets

(11) EP 3 247 787 B1

(12) **EUROPEAN PATENT SPECIFICATION**

(45) Date of publication and mention of the grant of the patent:
**12.10.2022 Bulletin 2022/41**

(21) Application number: **16701476.0**

(22) Date of filing: **22.01.2016**

(51) International Patent Classification (IPC):
***C12N 1/14*** *(2006.01)* ***A01H 15/00*** *(2006.01)*
***A01H 5/00*** *(2018.01)* *C12R 1/645* *(2006.01)*

(52) Cooperative Patent Classification (CPC):
**C12N 1/14; A01H 15/00; C12N 1/145;**
C12R 2001/645

(86) International application number:
**PCT/EP2016/051393**

(87) International publication number:
**WO 2016/116632 (28.07.2016 Gazette 2016/30)**

(54) **NEW HYBRID AGARICUS BISPORUS MUSHROOMS STRAINS**

NEUE HYBRIDE STÄMME VOM AGARICUS BISPORUS-PILZ

NOUVELLES SOUCHES DE CHAMPIGNONS AGARICUS BISPORUS HYBRIDE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.01.2015 EP 15305073**

(43) Date of publication of application:
**29.11.2017 Bulletin 2017/48**

(73) Proprietor: **Somycel**
**37130 Langeais (FR)**

(72) Inventors:
- **RODIER, Anne**
**49400 Saumur (FR)**
- **RUCKLIDGE, Richard**
**Worthing**
**West Sussex BN14 9EB (GB)**
- **BITAUDEAU, Stéphanie**
**37130 Mazieres De Touraine (FR)**

(74) Representative: **Regimbeau**
**20, rue de Chazelles**
**75847 Paris Cedex 17 (FR)**

(56) References cited:
**US-A1- 2010 154 079**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 247 787 B1

SEQUENCE LISTING

<110> SOMYCEL

<120> NEW HYBRID AGARICUS BISPORUS MUSHROOMS STRAINS

<130> B71654D34403

<150> EP 15305073.7
<151> 2015-01-23

<160> 17

<170> PatentIn version 3.5

<210> 1
<211> 1114
<212> DNA
<213> artificial

<220>
<223> P1N150 Allele 1

<220>
<221> misc_feature
<223> P1N150 Allele 1

<220>
<221> misc_feature
<222> (1114)..(1114)
<223> n is a, c, g, or t

<400> 1

```
taaccgagac tgtgctctcg taccaagcaa cctgggaatc accctgattt gcgttcattt        60
taactagatt agctgccatc tcaattcggt cggtcaaagg cacgtactct tctcgtatct       120
tttctccctt cgtcacgaga gcgctatact ctcgtcgaga cagaccatct agcagattcc       180
ctatcaaatt actgtagcat tgctcccaag ttgtatacca gccattcctc tgacgcgttg       240
ctccacaaaa agtcggaacc tcaaacccaa agccctcgtg gctcttgtac tgatgtaact       300
cagttgccag cctcttcgct agaagatcgg aggcacccga agacagaggc gccatatcct       360
tgtattccga aacaaagaaa ggcgtcccat cttcaccgtt accgtaggca atcatcctca       420
gtaaaaagag ttttcaggca ttttcgggta tctcaggccc tgaacatagg ctgagaattg       480
tcctctttct cgaacaatgc gagaacatga tcagccaaag ttcaggcaag ttgctttaaa       540
tataatacat ttgttgggcc aagctcaggc tgtgttcagg catcgttcaa ggtgtaagaa       600
cttcaactgt caccgacctt cagggtgcca atcgacccta aatcgaccct aaatcagcct       660
gcattatcca gaattacgtt gaattgacct ggattagcct gatagatcct gataatattt       720
tttactgaga atcatcgctg gcgcaagccc aggagctgcg gtacctatgg cttcgagtga       780
tcgcgcttcg ccaagatact gctcagactc cgacggcgat cctgatttca cgaaatacgt       840
ttgaccagtt gatgactgaa ccttgggaag atttccggtg aactgagcat ctggatcgat       900
gctttgcaga ttttcgagta tgatacgagg tagcattgga attgggaaag gtttagaatt       960
tctcaccgcg gagctccgga tcgtgctaat aatagtgacg tcagtgaacc tatcacggca      1020
gtccgtcgtc gaactttggt cgaaccgaca ttccacgttt cccatccagt tctttttttc      1080
atcccatgct gacaaatagc cgtggtccaa cacn                                  1114
```

<210> 2
<211> 877
<212> DNA
<213> artificial

<220>
<223> P1N150 Allele 2

<220>
<221> misc_feature
<223> P1N150 Allele 2

<220>
<221> misc_feature
<222> (1)..(1)
<223> n is a, c, g, or t

<220>
<221> misc_feature
<222> (5)..(5)
<223> n is a, c, g, or t

<220>
<221> misc_feature
<222> (876)..(877)
<223> n is a, c, g, or t

<400> 2

```
ncaangcttg ccctggaatc atagtcaaga aargggacat aatcgagact gtactctcgt      60
accaagcaac ctgggaatca ccctgatttg cgttcatttt aactagatta gctgccatct     120
caattcggtc ggtcaaaggc acgtactttt ctcgtatctt ttctcccttc gtcacgagag     180
cgccatactc tcgtcgagac aggccatcta gcagattccc tatcaaatta ctgtagcatt     240
gctcccaagt tgtataccag ccattcctct gacgcgttgc tccacaaaaa gtcggaacct     300
caaacccaaa gccctcgtgg ctcttgtact gatgtaactc agttgccagc ctcttcgcta     360
gaagatcaga ggcacccgaa gacagaggcg ccatatcctt gtattccgaa acaaagaaag     420
gcatcccatc ttcaccgtta ccgtaggcaa tcatcgctgg cgcaagccca ggagctgcgg     480
tacctatggc ttcgagtgat cgcgcttcgc caagatactg ctcagactcc gacggcgatc     540
ctgatttcac gaaatacgtt tgaccagtcg atgactgaac cttgggaaga tttccggtga     600
actgagcatc tggatcgatc ctttgcagat tttcgagtat gatacgaggt agcattggaa     660
ttgggaaagg tttggaattt ctcaccgcgg agctccggat cgtgctaata atagtgacgt     720
cagtgaacct atcacggcag tccgtcgtcg aactttggtc gaaccgacat tccacgtttc     780
ccatccagtt atttttttca tcccatgctg acaaatagcc gtggtccaac acctataaaa     840
acgacctcgc tcagcatgcg cttctgacat gtcacnn                              877
```

<210> 3
<211> 545
<212> DNA
<213> artificial

<220>
<223> P1N150 Allele 3

<220>
<221> misc_feature
<223> P1N150 Allele 3

<400> 3

atgaaaataa ctggatggga aacgtggaat gtcggttcga ccaaagttcg acgacggact 60

gccgtgatag gttcactgac gtcattatta ttagcacgat ccggagctcc gcggtgagaa 120

attccaaacc tttcccaatt ccaatgctac ctcgtatcat actcgaaaat ctgcaaagga 180

tccatccaga tgctcagttc accggaaatc ttcccaaggt tcagtcatca actggtcaaa 240

cgtatttcgt gaaatcagga tcgccgtcgg agtctgagca gtatcttggc gaagcgcgat 300

cactcgaagc cataggtacc gcagctcctg ggcttgcgcc agcgatgatt gcctacggta 360

acggtgaaga tgggatgcct ttctttgttt cggaatacaa ggatatggcg cctctgtctt 420

cgggtgcctc tgatcttcta gcgaagaggc tggcaactga gttacatcag tacaagagcc 480

acgagggctt tgggtttgag gttccgactt tttgtggagc aacgcgtcag aggaatggct 540

ggtat 545

<210> 4
<211> 598
<212> DNA
<213> artificial

<220>
<223> P1N150 Allele 4

<220>
<221> misc_feature
<223> P1N150 Allele 4

<400> 4

tcccaagttg tataccagcc attcctctga cgcgtcgctc cacaaaaagt cggaacctca 60

aatccaaagc cctcgtggct cttgtactga tgtaactcag ttgccagcct cttcgctaga 120

agatcagagg cacccgaaga caaaggcgcc atattcttgt attccgaaat aaagaaaggc 180

gccccatctt caccgttacc gtaggcaatc atcgctggcg caagcccagg agctgcggta 240

cctatggctt cgagtgatcg cgcttcgcca agatactgct cagactccga cggcgatcct 300

gatttcacga aatacgtttg accagttgat gactgagctt tgggaagatt tccggtgaac 360

tgagcatccg gatcgatcct ttgcagattt tcgagtatga tacgaggtag cattggaatt 420

gagaaaggtt tggaatttct caccgcggag ctccggatcg tgctaataat agtgacgtca 480

gtgaacctat cacggcagtc cgtcgtcgaa ctttggtcaa accgacattc cacgtttccc 540

atccagtact ttttttcatc ccatgctgac aaatagccgt agtccaacac ctataaaa 598

<210> 5
<211> 558
<212> DNA
<213> artificial

<220>
<223> P1N150 Allele 7

<220>
<221> misc_feature
<223> P1N150 Allele 7

<400> 5

```
aatccaaagc cctcgtggct cttgtactga tgtaactcag ttgccagcct cttcgctaga      60
agatcagagg cacccgaaga caaaggcgcc atattcttgt attccgaaat aaagaaaggc     120
gccccatctt caccgttacc gtaggcaatc atcgctggcg caagcccagg agccgcggta     180
cctatggctt cgagtgatcg cgcttcgcca agatactgct cagactccga cggcgatcct     240
gatttcacga aatacgtttg accagttgat gactgagctt tgggaagatt tccggtgaac     300
tgagcatccg gatcgatcct ttgcagattt tcgagtatga tacgaggtag cattggaatt     360
ggtaaaggtt tggaatttct caccgcggag ctccggatcg tgctaataat agtgacgtca     420
gtgaaactat cacggcagtc cgtcgtcgaa ctttggtcaa accgacattc cacgtttccc     480
atccagttct tttttccatc ccatgctgac aaatagccgt agtccaacac ctataaaaac     540
gacctcgaat ccaaagcc                                                   558
```

<210> 6
<211> 18
<212> DNA
<213> artificial

<220>
<223> forward primer

<400> 6
aggcryccca tcttcasc 18

<210> 7
<211> 17
<212> DNA
<213> artificial

<220>
<223> reverse primer

<400> 7
gttcgacgac ggactgc 17

<210> 8
<211> 19
<212> DNA
<213> artificial

<220>
<223> forward primer

<400> 8
tccgtaggtg aacctgcgg 19

<210> 9
<211> 20
<212> DNA
<213> artificial

<220>
<223> reverse primer

<400> 9
tcctccgctt attgatatgc 20

<210> 10
<211> 23
<212> DNA
<213> artificial

<220>
<223> forward primer

<400> 10
aytcrcaama acataccttc aac 23

<210> 11
<211> 18
<212> DNA
<213> artificial

<220>
<223> reverse primer

<400> 11
cattcggcga ttttctca 18

<210> 12
<211> 21
<212> DNA
<213> artificial

<220>
<223> forward primer

<400> 12
gacgatgcgg gactggtgga t 21

<210> 13
<211> 23
<212> DNA
<213> artificial

<220>
<223> reverse primer

<400> 13
ggtctggcct acrggagtgt tgt 23

<210> 14
<211> 23
<212> DNA
<213> artificial

<220>
<223> forward primer

<400> 14
ccgccagcac aaggaatcaa atg 23

<210> 15
<211> 22
<212> DNA
<213> artificial

<220>
<223> reverse primer

<400> 15
tcagtcggcc ctcaaaacag tc 22

<210> 16
<211> 22
<212> DNA
<213> artificial

<220>
<223> forward primer

<400> 16
tcgggtggtt gcaactgaaa ag 22

<210> 17
<211> 24
<212> DNA
<213> artificial

<220>
<223> reverse primer

<400> 17
ttcctttccg ccttaattgt ttct 24

## Description

### Technical field

The present invention relates to the field of microorganism strain development, more particularly to the development of heterokaryotic hybrids of the fungus species *Agaricus bisporus*. Even more particularly, the present invention relates to white *Agaricus bisporus* hybrid strains having improved characteristics for the users (namely growers, processors and consumers). These hybrids are for example the deposited strains CNCM I-4945 and 1-4946, originating from a cross plan involving the deposited line CNCM I-4948. The present invention relates to strains derived from these hybrids and also progenies derived from these hybrids.

### Background of the invention

*Agaricus bisporus* (Lange) is a microorganism (kingdom: Fungi, phylum: Basidiomycota, class: Agaricomycetes, order: Agaricales, family: Agaricaceae, genus: *Agaricus,* section: Bivelares, species: *bisporus*). This microorganism remains the most widely cultivated edible fungal species accounting for 38% of the world production (International Society for Mushroom Science (ISMS) website). Mushrooms are mainly sold as fresh vegetables or processed (canned, pasteurised or frozen). They are interesting components of diet due to the interesting taste, aroma and nutritional values (proteins, indigestible fibres, vitamins, minerals, amino-acids) (Beelman et al., 2008). They also have medicinal effects including control of cancers and metabolic diseases (Chen & Kanaya, 2012).

Nowadays white mushroom consumption worldwide contributes to a multibillion dollar industry (Morin et al., 2012). More precisely, the worldwide production of mushrooms reached 7,959,979 tons in 2013 (FAO stat, 2013).

As mushrooms are grown under controlled environment indoors all year round in exclusively dedicated facilities requiring heavy investment, any gain on productivity or efficiency (profitability) is welcome by the industry. An increase of the crop yield or decrease in the growing cycle length can positively impact the turnover of the business. More generally, a combination of improvement for both factors can potentially have a very significant financial impact and would therefore be economically desirable.

The first real hybrid strains of *A. bisporus* generated by true breeding in the 1980s in Holland (Horst U1 = Horronda and Horst U3 = Horwitu) have been very successful commercially and have had a major impact on the production and marketing of the button mushroom. To date, most if not all commercial varieties of *A. bisporus* var. *bisporus* with white caps are actually copies (clones) or derivatives of Horst U1 (Foulongne-Oriol et al., 2011, Sonnenberg et al., 2011) and *A. bisporus* appears to be a monolineage crop (Savoie et al., 2013), at least for the white strains. This is especially relevant in territories such as the European countries, where white capped commercial varieties are currently clearly dominant on the market. In 2013 almost all of the white commercial strains showed a striking similarity to Horst U1 at the genetic level (Savoie et al., 2013).

However the lack of diversity in the cultivated strains of the mushroom is viewed as an important risk and threat for this culture. As a matter of fact, due to the lack of genetic diversity among commercial strains at the moment, no real differences regarding agronomic, quality or processing performance of mushrooms can be expected when comparing them under the same conditions. Moreover the lack of genetic diversity among commercial strains generates an important risk of genetic erosion (sudden miss-adaptation in the context of production, cropping or usage) with consequences in terms of risk management, business risk and continuity of supply. Thus the absence of initial diversity raises sanitary and economic risks. In this context several companies have initiated breeding programs aimed at obtaining new genetic material able to offer new benefits and opportunities (diversification, segmentation) to the users (growers, processors, consumers).

Several efforts to breed new white varieties and introduce them to the market have been attempted (US 5,832,659, US 8,663,969), however with little success. One of the hurdles to the development of white strains is that a very high percentage of the wild diversity carries the brown cap colour, which expresses positive partial dominance with a complex genetic control involving a major locus and several minor loci (Foulongne-Oriol et al., 2012). As a consequence, at least two generations are required to bring back the white cap colour into the breeding material. Other characters must also be taken into account, such as the reaction to bruising (Gao et al., 2013), the shelf-life, etc. The goal of launching white strains therefore appears more complicated compared to brown strains.

Two different *Agaricus bisporus* botanical varieties expressing different life cycles compared to *A. bisporus* var. *bisporus* have been identified based on biology, phenotype and genetics. *A. bisporus* var. *burnettii* (Callac, 1993) and *A. bisporus* var. *eurotetrasporus* (Callac et al, 2003) have respectively predominantly heterothallic and homothallic life cycles. This makes it possible to obtain homokaryons more easily due to a higher percentage recovery from a spore print (Kerrigan et al. 1994). However, though they exhibit inter-fertility with *A. bisporus* var. *bisporus,* their use in breeding strategies has proven disappointing due to major phenotypical drawbacks genetically transmitted to their offspring, requiring subsequent generations in order to remedy and return back to a commercially acceptable phenotype.

Thus the market of white mushrooms and more specifically of *A. bisporus* mushrooms, looks forward to innovations allowing improvement and diversification at any stage of the production process. These innovations may help producer profitability (higher yield, shorter cycle time, earlier harvest, greater ease and speed of harvesting etc.) and/or the demand in the distribution chain (improved visual appeal, improved or distinct flavour characteristics, longer persistence of desirable visual attributes, etc.). In particular, it would be advantageous to generate mushrooms strains having improved first-break appearance, e.g. less pronounced cap scaling and/or a whiter appearance. Still other improvements may enhance the suitability of the mushroom crop for mechanical harvesting, canning and/or food processing. Finally it is important to identify virus-breaking varieties, i.e. vegetatively incompatible mushrooms, in order to avoid transmission of viral diseases in mushroom growing facilities.

The present invention fulfils this need by providing new hybrid strains of white *A. bisporus* mushroom cultures exhibiting a number of improved characteristics for producer and consumer profitability.

By screening the agronomic value, the phenotype value and the canning yield of a number of white *A. bisporus* mushroom progenies, the inventors indeed identified interesting hybrids having i) a high average weight and ii) an appropriate phenotype for mechanical harvesting (individual growth of mushrooms, good distribution on the crop surface, straight stipe) and/or for the consumer (smooth white-cap colour) and iii) an enhanced canning yield and agronomic value (including in large scale trials), as compared to current commercial varieties. Moreover these hybrids are vegetatively incompatible with current commercial white strains. In other words, these hybrids of the invention exhibit a combination of enhanced agronomic value and an appropriate phenotype for producer, processor and consumer satisfaction.

## Detailed description of the invention

Initially, in orderto provide a clear and consistent understanding of the specification and claims, the following information and definitions are provided.

### *Mushrooms structure*

Mushrooms are composed of filaments (hyphae) compartmentalised into articles (cells). In contrast to vegetative mycelium, sporophore hyphae are differentiated into distinct tissue forming a network of hyphae interlaced, with different orientations, diameters and densities when comparing different areas across and within the stipe and the cap (Rama et al. 1997, Rama et al. 2000). Air spaces between hyphae of the cap represent up to 50 % of the total volume, with a higher percentage in the upper layers and a lower percentage in the deeper layers. This percentage is lower in the gills, around 25 %, comparable to the stipe (Donker et al., 1996). *A. bisporus* **fruiting bodies** are reproductive differentiated parts of macroscopic fungi and **sporophores** are sold under the name of **button mushroom.**

As used herein, the term **"mushroom"** designates, depending on the context in which it is used, the reproductive structure of an Agaric fungus or an Agaric or a cultivated food product of the same name.

**"Spores"** are the reproductive propagule of the mushroom. Living spores are either heterokaryons or homokaryons in a dormant state. Spores may be aseptically collected on sterile material, suspended in sterile liquid medium, diluted and plated onto sterile agar growth media in orderto produce germinated spores and the cultures incorporated within the spores. Different techniques may stimulate spore germination via the diffusion of a volatile pheromone: introduction of a living *Agaricus* culture in the lid of the plate grown upside-down, or on a side of the plate, or the use of isovaleric acid. Germinated spores may be isolated under a dissecting microscope using sterile micro-tools such as steel dissecting needles or scalpel blades, onto fresh nutrient agar plates. Using this method, heterokaryotic and homokaryotic offspring of a heterokaryotic strain comprising the spores and the cultures incorporated within the spores may be obtained.

Other parts of the mushroom include caps, stems, gills, cells (defined as hyphal compartments incorporating nuclei, mitochondria, cytoplasm, a cell membrane and a cell wall including cross-walls), hyphae and mycelium.

### *Mushroom breeding*

A complete review of genetics and genomics of cultivated mushrooms applied to the breeding of Agarics, including germplasm and genetic improvement, has been published recently (Savoie et al., 2013).

As other fungal species *A. bisporus* has different reproduction processes: vegetative reproduction (mitosis), sexual reproduction (meiosis) and parasexual reproduction (other phenomena). Most *Basidiomycete* fungi produce four spores per basidium, each of them receiving one nucleus and each of these spores will germinate as a haploid mycelium called a homokaryon, able to mate to another homokaryon.

The typical life cycle of *A. bisporus* is amphithallic (pseudohomothallic, secondary homothallic), or heterothallic, depending on the level of ploidy of the spores, which can be respectively heterokaryotic ($n+n$ chromosomes) or homokaryotic ($n$ chromosomes).

As used herein the term **"homokaryon"** designates a haploid culture with a single type (or somatic lineage) of haploid nucleus (cytogenetically represented as *n*). It is ordinarily reproductively incompetent (i.e. does not fruit) but may function as a gamete in sexually complementary anastomoses. Though haploid, homokaryons are also referred to as **"lines"** which transmit a uniform genotype to offspring.

On the other hand **"heterokaryon"** refers to a culture which has two complementary types of haploid nuclei in a common cytoplasm. It is thus functionally and physiologically analogous to a diploid individual (but cytogenetically represented as *n+n* rather than 2n). Unlike homokaryons, it is reproductively competent (i.e. able to fruit). Heterokaryons are also called **"strains"** in the breeding context. In the context of the invention heterokaryotic strains produced from controlled matings are also called **"hybrids"**.

For *A. bisporus* var. *bisporus,* breeding is more complicated compared to other fungi species due to a specific life cycle.

*A. bisporus* var. *bisporus* carries a majority of bisporic heterokaryotic basidia producing fertile heterokaryons characterising a predominant pseudohomothallic life cycle. The heterothallic cycle produces homokaryons. *A. bisporus* var. *bisporus* has a multi-allelic unifactorial system of sexual inter-compatibility, under control of the *MAT* (Mating Type) locus characterised by a series of 14 Mat alleles. However two homokaryons can mate (plasmogamy) and produce a hybrid heterokaryotic fertile mycelium only if they are sexually compatible, i.e. they carry different alleles. This phenomenon is taken into account by breeders to design breeding schemes and cross plans. However since only a small percentage of spores produce homokaryons this complicates and slows breeding work.

As used herein the term **"anastomosis"** designates the fusion of two or more hyphae that achieves cytoplasmic continuity. Moreover the term **"plasmogamy"** refers to the establishment, via anastomosis, of cytoplasmic continuity leading to the formation of a sexual heterokaryon with exchange of nuclei, mitochondria, cytoplasm and other intracellular components. The sexual union of two cultures via anastomosis and plasmogamy is herein referred to as **"mating".**

The term **"mushroom culture"** herein designates the mushroom microorganism propagated on various growth media and substrates. It refers to a physical strain, line, homokaryon or heterokaryon. Once obtained, a culture can be vegetatively reproduced at laboratory level.

***Growing process of A. bisporus***

*A. bisporus* is a saprophytic species and leaf-litter secondary decomposer. It is grown on compost prepared from agricultural by-products or waste (e.g. straw) on occasions supplemented with animal manures (horse, chicken) and other additives (calcium source e.g. gypsum). Growing systems across the world range from small labour intensive family farms in developing countries to high investment industrialised automated systems. In the most modern systems, for each step of the process, from spawn making to compost production, growing, harvesting and processing, specialised companies are involved.

*A. bisporus* is a micro-organism that can be handled by classical microbiology techniques in laboratories under sterile conditions (laminar hood and/or clean area, sterile media, instruments, equipment, packages etc.). Its optimum conditions are temperature approximately 24 °C, pH approximately 7. Mycelium or spores can be stored in a cool room (e.g. 2 °C). Growing media can be malt extract agar, potato dextrose agar, potato dextrose yeast agar, specific *Agaricus* compost agar, etc.

**Mushroom spawn** consists of a carrier (usually cooked cereals or synthetic products) inoculated in aseptic or sterile conditions with a pure culture of a strain. In other terms, **"spawn"** herein refers to a mushroom culture, typically a pure culture of a heterokaryon, carried by a sterile substrate which is friable and dispersible particulate matter (e.g. cereal grains).

More precisely the growing process starts by mixing spawn into compost (spawning) followed by a vegetative phase (spawn run or phase III). Inoculated compost is incubated for 13 to 19 days under a controlled environment to maintain optimum temperature, $CO_2$ concentration and relative humidity in the air. During this phase the mycelium feeds from the compost, develops biomass and colonizes the medium. As a result the metabolic heat generated by the compost increases until full colonisation and then decreases. The pH decreases from approximately 7.5 to 6 and the colour of the medium changes from brown (compost) to white (mycelium).

Subsequently a layer of inert material known as casing (e.g. pH adjusted peat or soil or mix e.g. peat and limestone) is added to the surface of the compost. In some instances mycelium is mixed with casing (either proprietary products or colonised compost) in order to promote colonisation of the casing layer and fructification. The growing parameters are similar to the spawn-run.

In the context of mushroom breeding the **"casing layer"** designates a layer of non-nutritive material (peat, soil or mix) that is applied to the upper surface of a mass of colonised compost in order to permit development of the mushroom crop. **"Casing inoculum"** (CI) designates the inoculum material incorporating a mushroom culture, typically of a defined heterokaryotic strain, suitable for mixing into the casing layer.

An adjustment of growing conditions triggers the change from vegetative to reproductive phase and fructification.

This generally involves a controlled decrease of temperature and relative humidity together with the introduction of fresh air (airing) to reduce the $CO_2$ level. Techniques of physically ruffling the surface, watering and chemical stress may also be involved at this stage.

Mushrooms generally develop within 7 to 14 days from airing to first harvest. The stage of picking or harvesting relies on the physiological development of the mushroom and is adjusted to the requirements of the grower for his market.

After picking or harvesting other mushrooms start growing as successive groups at approximately weekly intervals. The period of mushroom production within a cropping cycle, separated by intervals of non-production is herein called a **"flush".** Successive flushes generally produce decreasing yield values. Two or three flushes are usual nowadays in industrial systems, more in traditional ones.

**Earliness** (timing) is usually expressed as the time from casing to picking. Between flushes the growing conditions (climate, watering) are also carefully managed in order to generate the appropriate number and quality of mushrooms and adjust the time of picking subsequent flushes.

Finally the crop is removed, spent compost is discarded, the facility cleaned and a new crop cycle commences.

Most modern facilities receiving phase III compost (spawn-run completed) and growing for 2 flushes have many growing rooms and can complete up to 13 successive growing cycles per room per year.

For the fresh market mushrooms are hand-picked, whereas for processing they are mechanically harvested. A harvest period over 3 to 5 days (staggering) is needed for hand picking, whereas evenness is required for mechanical harvesting (single operation). This is obtained by respectively a smooth transition from vegetative to reproductive phase, or a quicker transition, which has an impact on the number and correlatively on the size of mushrooms.

Humidity of the casing, appropriate management of picking and hygiene to avoid pests and diseases are also key factors in crop management in order to ensure quantity (yield) and quality at picking or harvesting. Longer crop cycles increase the risk of epidemics of pests and diseases. First flushes are always cleaner than second or even third flushes.

**Crop yield** is defined as the mushroom weight picked (or harvested) per unit of surface area or more specifically to the mushroom weight picked (or harvested) per weight of pasteurised compost i.e. as a percentage.

***Mushrooms processing after harvesting***

**Processing** is aimed at preserving food such as vegetables by extending its shelf-life by canning, pasteurising, freezing, freeze-drying, drying for example. This is of particular interest for mushrooms due to a naturally short shelf-life (around 5 days after picking), especially when supply exceeds demand.

Processing is organised into several steps. During canning, evacuation consists of placing mushrooms under vacuum conditions following a pre-treatment with water and additives, so that the air is evacuated and replaced by liquid. Blanching consists of submerging mushrooms into hot or boiling water with additives: heating makes them less susceptible to deterioration. Mushrooms can be preserved whole or sliced. In the last step, mushrooms can be pasteurised or sterilised. Pasteurisation consists of heating the mushrooms to 95 °C for an extended shelf-life (months if refrigerated), whereas sterilisation consists of autoclaving the mushrooms up to approximately 125 °C for an extended shelf-life (years at room temperature). Cooling and soaking can occur at different stages of the process. Containers can be cans, jars, bags etc.

The efficiency of the canning process can be calculated. **Canning yield** is the ratio of weight of canned product at the end of the process compared to the weight of fresh product introduced at the beginning and can be expressed as a ratio, a percentage, etc. % canned product yield = (total drained weight / initial sample weight) x 100 (Beelman et al. 1973).

Shrinkage, i.e. loss of processed product weight occurring during blanching and thermal processing, results in a decrease of the canning yield. Since the canned product is sold on a drained-weight basis, excessive shrinkage results in considerable economic loss to processors. A study performed on costs and revenues calculated that for a medium size plant a 5% reduction in shrinkage would increase revenues by 20% and hourly revenues above costs by 21.8% (Coale et al. 1972). Of note, off-white strains (such as Somycel 76) are characterised by a higher loss of weight after canning (i.e. a lower CY) as compared to smooth-white strains. Moreover, they provide a lower quality (the cap tissue is less white, the gills turn brown earlier) (Steinbuch 1978, Fristche, 1983).

Many factors are deemed to have an impact on the canning yield: grade of the mushrooms (cap diameter), stage (physiological maturity), flush, cropping techniques, storage conditions (time and climate parameters) etc. and also technological process parameters. Due to the large volumes processed, optimisation of raw materials and processes is of first concern to canneries and gains of canning yield are welcome by the industry, especially when they have commodity margins. The lack of genetic diversity in current commercial strains restricts the field of improvement and gain to technological process parameters.

Crop yield is important both for fresh and processing markets and processing yield is most important for the industry preserving mushrooms.

In modern systems, crop yield and processing yield are combined into a calculation of the financial value of the crop, i.e. the financial outcome for example crop value x processing value. An improvement of the crop yield or of the processing yield can have an impact on the financial outcome and as a consequence, a combination of a gain for both of these parameters can potentially have a very significant financial impact. The financial efficiency can be calculated as the drained weight of processed mushrooms vs. mass of pasteurised substrate at spawning (%) or as drained weight of processed mushrooms/$m^2$ of the crop.

***Development of progenies***

To develop a progeny from a given strain, the first step is to obtain a spore print from a mushroom of this strain. A mushroom is picked from a crop at the appropriate stage (as the veil stretches but before it breaks) and placed in an aseptic chamber (e.g. beaker) above a sterile sheet (e.g. autoclaved tracing paper) at room temperature. The mushrooms will mature within a few days and the spores will be released onto the paper. Spore prints can be preserved at fridge temperature once packed in a sterile plate or tube.

The following steps are completed in a sterile environment (laminar hood, clean area). Petri dishes of an appropriate medium are prepared, with either a mycelium of A. *bisporus* or isovaleric acid, both having the property of stimulating the germination of spores.

In a second step, a solution of spores is prepared, plated on the petri dishes prepared previously and allowed to grow for a few days (incubator).

In a third step, as soon as germination starts, individual spores (just germinated) are picked (dissecting microscope under a sterile hood) using sterile instruments (e.g. dissecting needles), transferred individually to new plates and grown (incubator).

***Vegetative incompatibility***

Vegetative compatibility/incompatibility (also referred to as **"heterokaryon incompatibility"** or "self/non-self recognition") is a phenomenon different from sexual compatibility. It is expressed at the heterokaryotic stage as an antagonistic phenomenon that prevents the mycelium of two distinct heterokaryons from anastomosing and creating continuity of cytoplasm. When grown side by side two vegetatively incompatible heterokaryons will show a delay in growth and/or in development of mushrooms, whereas two compatible heterokaryons will show a normal growth and timing.

Vegetative incompatibility can be an advantage in terms of the hygiene of mushroom growing facilities. A range of viral diseases can infect mushroom crops, most common being Virus X (MVXD) and La France Virus (LIV) (Fletcher & Gaze, 2008). Two possible means of virus transfer are known:

- From mycelium to mycelium through hyphal fusion (anastomosis) with the transfer of cell content,
- In mushroom spores which germinate and pass the virus to healthy mycelium by anastomosis.

Anastomosis between mushroom strains is essential for virus transfer. Epidemics develop through fragments of viable mycelium left on or in equipment or through airborne fragments when handling incubated bulk compost (during or at the end of the spawn-run, at ruffling or casing ...). In this way the disease can be spread within a facility or between facilities.

Once a farm is infected the use of a second alternate strain exhibiting vegetative incompatibility to the usually grown strain can break the cycle of infection. In this case, the second strain is deemed to have virus-breaking properties.

It is known that within the mid-range hybrids (derivatives of U1) anastomosis occurs, i.e. strains are vegetatively compatible. Conversely off-white strains do not readily anastomose with smooth-whites i.e. these strains are vegetatively incompatible (Fletcher & Gaze, 2008).

***Hybrids of the invention***

The present invention is directed to an *Agaricus bisporus* hybrid strain having the strain J10117 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, as first parent and an *Agaricus bisporus* white strain as a second parent, said hybrid strain being selected in the group consisting of:

- LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4945,

- LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946, and
- Derivatives thereof wherein said derivatives are *Agaricus bisporus* hybrids comprising all the SSR molecular markers and/or P1N150 allelic sequences of the hybrid **LB811** having been deposited under the CNCM Accession Number I-4945 or of the hybrid **LB906** having been deposited under the CNCM Accession Number I-4946 or of the hybrid **J10117** having been deposited under the CNCM Accession Number 1-4950,

wherein the P1N150 allelic combinations for LB811, LB906 and J10117 are the ones as in Table 17,

and wherein the SSR molecular markers for LB811, LB906 and J10117 are the ones as in Table 15bis,

wherein the said hybrid strain has an improved canning yield, the improved canning yield being defined as a higher canning yield compared to mushroom from a control commercial *Agaricus bisporus* strain A15.

. These hybrids are hereafter referred to as **"J10117"** (or "J 10117"), **"LB811"** (or "LB-811") and **"LB906"** (or "LB-906").

A deposit of a culture of the *Agaricus bisporus* hybrid **J10117** has been made with the Collection Nationale de Cultures de Microorganismes (CNCM) of the Institut Pasteur, 25, rue du Docteur Roux, F-75724 PARIS CEDEX 15, FRANCE. The date of deposit was January 20, 2015. Said deposit was made by SOMYCEL, prior to the filing date of this application. The deposit is intended to meet all deposit requirements under the Budapest Treaty. The CNCM Accession No. is **I-4950.** The deposit will be maintained in the depository for a period of 30 years or 5 years after the last request or for the effective life of the patent, whichever is longer and will be replaced as necessary during that period.

The *Agaricus bisporus* hybrid **J10117** can be crossed with any *Agaricus bisporus* strain. Said strain is for example an *A. bisporus* white strain or a brown strain.

In a preferred embodiment, said white strain is U1 or a derivative thereof, or any usefully equivalent white strain (i.e. either identical with, belonging to the same derived lineage group as, or functionally similar to). Other white strains include traditional off-white (=OW) strains such as, for example, Somycel 9.2, Somycel 76 or Somycel 611 or Smooth-White (=SW) strains such as, for example, Somycel 53. These and other OW strains form a derived lineage group which share a common genetic identity and are functionally equivalent and interchangeable. A deposit of a culture of hybrid strain Somycel 76, as disclosed herein, has been made with the Agricultural Research Services Culture Collection (NRRL) 1815 North University Street, Peoria, Illinois 61604 USA on February 18, 2014, under the NRRL Accession No. 50903. In another preferred embodiment, said white strain is **Fungisem FS 9.18,** that is commercially available.

In a preferred embodiment, said brown strain is Sylvan 800 or Sylvan 856. Homokaryotic lines have been obtained from the hybrids J10117, Somycel 76 and FS9-18, and hybrid progenies have been generated by conventional mating of these lines. Interestingly, two of them (namely **LB811** and **LB906)** exhibited improvement for the canning yield and the agronomic values (in terms of mushroom weight, cap size, timing, etc.) as well as an appropriate phenotype for producer and consumer satisfaction (lightness, smoothness, cap shape, stipe diameter, see examples below). Moreover, they have been shown to be vegetatively incompatible with all tested commercial strains, including their parents J10117, Somycel 76 and FS9-18.

**LB811** and **LB906** have J10117 as a first parent. More precisely the hybrid **LB811** has the strain J10117 as a first parent and the strain FS9-18 as the second parent. Moreover the hybrid **LB906** has the strain J10117 as a first parent and the strain Somycel 76 as the second parent.

In a first aspect the present invention relates to hybrid mushroom strains having the strain J10117 as a first parent and an *Agaricus bisporus* white or brown strain as a second parent. In a preferred embodiment the said white strain is U1 or derivative thereof or an OW-type strain or a SW-type strain. In a more preferred embodiment, said white strain is an OW strain selected in the group consisting of: Somycel 9.2, Somycel 76 or Somycel 611 and their derivatives. In another more preferred embodiment, said white strain is Fungisem FS 9-18.

Specifically, the homokaryotic lines **J10117s62, 76s14** and **FS9-18s50** have been obtained from the hybrids J10117, Somycel 76 and FS9-18 respectively. The hybrids LB811 and LB906 have been produced by mating the homokaryotic *Agaricus bisporus* line J10117s62 (obtained from J10117) with homokaryotic lines (76s14 and FS9-18s50) derived from the commercially available strains Somycel 76 and FS9-18 respectively. Advantageously, these two hybrids exhibited improvement for first break aspect, canning yield, agronomic yield and value (e.g. mushroom weight and size) and an appropriate phenotype for producer and consumer satisfaction (e.g. stipe diameter, lightness and smoothness of the cap, etc.).

In another aspect, the present description discloses a method of producing the hybrids of the invention (J10117, LB811 and / or LB906).

In a particular embodiment the said method comprises the steps of:

a) Obtaining spores from said hybrid mushroom culture,

b) Germinating said spores,

c) Transferring cultures from germinating spores to new growth media.

Each step can be performed by any conventional means, as disclosed above.

***CNCM deposits***

A deposit of a culture of the *Agaricus bisporus* homokaryon **J10117s62** has been made with the Collection Nationale de Cultures de Microorganismes (CNCM), Institut Pasteur, 25 rue du Docteur Roux, 75724 PARIS Cedex 15. The date of deposit was January 20, 2015. Said deposit was made by SOMYCEL, prior to the filing date of this application. The deposit is intended to meet all requirements under the Budapest Treaty. The CNCM Accession No. is **I-4948.** The deposit will be maintained in the depository for a period of 30 years, or 5 years after the last request, or for the effective life of the patent, whichever is longer, and will be replaced as necessary during that period.

A deposit of a culture of the *Agaricus bisporus* homokaryon **76s14** has been made with the Collection Nationale de Cultures de Microorganismes (CNCM), Institut Pasteur, 25 rue du Docteur Roux, 75724 PARIS Cedex 15. The date of deposit was January 20, 2015. Said deposit was made by SOMYCEL, prior to the filing date of this application. The deposit is intended to meet all requirements under the Budapest Treaty. The CNCM Accession No. is **I-4947.** The deposit will be maintained in the depository for a period of 30 years, or 5 years after the last request, or for the effective life of the patent, whichever is longer and will be replaced as necessary during that period.

A deposit of a culture of the *Agaricus bisporus* heterokaryon **FS9-18** has been made with the Collection Nationale de Cultures de Microorganismes (CNCM), Institut Pasteur, 25 rue du Docteur Roux, 75724 PARIS Cedex 15. The date of deposit was January 20, 2015. Said deposit was made by SOMYCEL, prior to the filing date of this application. The deposit is intended to meet all requirements under the Budapest Treaty. The CNCM Accession No. is **I-4949.** The deposit will be maintained in the depository for a period of 30 years, or 5 years after the last request, or for the effective life of the patent, whichever is longer and will be replaced as necessary during that period. This heterokaryon generated homokaryons such as FS9.18s50, which have been used to generate LB811.

In a preferred embodiment the hybrid of the invention is the *Agaricus bisporus* hybrid **LB811** which has been deposited with the Collection Nationale de Cultures de Microorganismes (CNCM) of the Institut Pasteur, 25, rue du Docteur Roux, F-75724 PARIS CEDEX 15, FRANCE on January 20, 2015 by SOMYCEL, and whose CNCM Accession No. is **I-4945.** This deposit is intended to meet all deposit requirements under the Budapest Treaty. In particular it will be maintained in the depository for a period of 30 years, or 5 years after the last request, or for the effective life of the patent, whichever is longer and will be replaced as necessary during that period.

In another preferred embodiment, the hybrid of the invention is the *Agaricus bisporus* hybrid **LB906** which has been deposited with the Collection Nationale de Cultures de Microorganismes (CNCM) of the Institut Pasteur, 25, rue du Docteur Roux, F-75724 PARIS CEDEX 15, FRANCE on January 20, 2015 by SOMYCEL, and whose CNCM Accession No. is **I-4946.** This deposit is intended to meet all deposit requirements under the Budapest Treaty. In particular it will be maintained in the depository for a period of 30 years, or 5 years after the last request, or for the effective life of the patent, whichever is longer and will be replaced as necessary during that period.

A brief description of the genotype of the hybrids of the invention at 22 SSR (Single Sequence Repeat) markers is provided below (for detail on these markers, see Foulongne-Oriol et al., 2009). Because the hybrids incorporate two sets of chromosomes, there are two allelic copies at each marker locus.

Table 1 presents a fingerprint of 22 SSR markers for the hybrids of the invention.

| markers | LB811 | LB906 | J10117 |
|---|---|---|---|
| AbSSR02 | 188/188 | 188/192 | 188/188 |
| AbSSR05 | 329/332 | 329/332 | 329/332 |
| AbSSR12 | 171/171 | 171/171 | 171/174 |
| AbSSR13 | 212/212 | 212/212 | 212/212 |
| AbSSR14 | 167/167 | 167/167 | 167/167 |
| AbSSR19 | 191/191 | 191/191 | 191/191 |
| AbSSR23 | 176/176 | 176/176 | 176/176 |

(continued)

| markers | LB811 | LB906 | J10117 |
|---|---|---|---|
| AbSSR31 | 167/167 | 167/167 | 167/167 |
| AbSSR33 | 192/192 | 192/192 | 192/192 |
| AbSSR36 | 149/157 | 149/149 | 149/149 |
| AbSSR39 | 186/188 | 186/188 | 186/188 |
| AbSSR42 | 177/179 | 179/179 | 177/179 |
| AbSSR43 | 231/231 | nd | 231/231 |
| AbSSR45 | 193/203 | 193/203 | 193/203 |
| AbSSR49 | 174/174 | 174/174 | 174/174 |
| AbSSR56 | 225/225 | 225/225 | 225/225 |
| AbSSR57 | 257/257 | 257/257 | 257/257 |
| AbSSR58 | 165/169 | 167/169 | 169/169 |
| AbSSR59 | 197/201 | 197/201 | 197/201 |
| AbSSR60 | 206/208 | 206/208 | 206/208 |
| AbSSR64 | 104/104 | 104/104 | 104/104 |
| AbSSR65 | 197/213 | 188/213 | 213/213 |

The marker profiles disclosed in Table I represent a small excerpt of the genome sequence of the claimed hybrids. These markers have been selected as they are distributed at widely spaced intervals spanning the entire nuclear genome and they are known to distinguish distinct mushroom commercial strains (Foulongne-Oriol et al., 2009).

For example, it is possible to distinguish the hybrid LB811 from all U1 derivatives by its genotype at any of the four markers AbSSR 23, 39, 58 and 65. Moreover, it is possible to distinguish the hybrid LB906 from all U1 derivatives by its genotype at any of the five markers AbSSR 02, 23, 36, 39, and 42. Finally, it is possible to distinguish the J10117 hybrid from all U1 derivatives by its genotype at any of the six markers AbSSR 12, 23, 36, 39, 58 and 65.

The hybrids and strains of the invention may be distinguished from commercial U1 derivative strains such as A15 as well as from other hybrid strains or homokaryon strains such as J10165 (as disclosed in US20100218294 and US2100154079), J11500 (as disclosed in WO/2015/114612) and J9277 (as disclosed in US20080182321 and US2010154079).

Table 2 represents a small excerpt of the genome sequence of the claimed hybrids versus known hybrids or strains and shows that, using the AbSSR markers as indicated above, there are many genetic differences between them.

Table 2 Fingerprinting of 22 SSR markers of the hybrids of the invention and of known strains

| markers | LB811 | LB906 | J10117 | | J10165 | J11500 | J9277 | | A15 |
|---|---|---|---|---|---|---|---|---|---|
| AbSSR02 | 188/188 | 188/192 | 188/188 | | 188/188 | 188/188 | 188/188 | | 188/188 |
| AbSSR05 | 329/332 | 329/332 | 329/332 | | 329/332 | -/- | 329/332 | | 329/332 |
| AbSSR12 | 171/171 | 171/171 | 171/174 | | 171/171 | 171/173 | 171/171 | | 171/171 |
| AbSSR13 | 212/212 | 212/212 | 212/212 | | 212/212 | 212/212 | 212/212 | | 212/212 |
| AbSSR14 | 167/167 | 167/167 | 167/167 | | 167/167 | 165/167 | 167/167 | | 167/167 |
| AbSSR19 | 191/191 | 191/191 | 191/191 | | 191/191 | 191/191 | 191/191 | | 191/191 |
| AbSSR23 | 176/176 | 176/176 | 176/176 | | 170/176 | 170/176 | 170/176 | | 170/176 |
| AbSSR31 | 167/167 | 167/167 | 167/167 | | 167/167 | 167/167 | 167/167 | | 167/167 |
| AbSSR33 | 192/192 | 192/192 | 192/192 | | 192/192 | 192/192 | 192/192 | | 192/192 |
| AbSSR36 | 149/157 | 149/149 | 149/149 | | 149/149 | 149/149 | 149/157 | | 149/157 |

(continued)

| markers | LB811 | LB906 | J10117 | | J10165 | J11500 | J9277 | | A15 |
|---|---|---|---|---|---|---|---|---|---|
| AbSSR39 | 186/188 | 186/188 | 186/188 | | 186/186 | 186/186 | 186/188 | | 186/186 |
| AbSSR42 | 177/179 | 179/179 | 177/179 | | 177/177 | 156/179 | 177/177 | | 177/179 |
| AbSSR43 | 231/231 | nd | 231/231 | | 231/231 | 231/231 | 231/231 | | 231/231 |
| AbSSR45 | 193/203 | 193/203 | 193/203 | | 193/203 | 193/203 | 193/197 | | 193/203 |
| AbSSR49 | 174/174 | 174/174 | 174/174 | | 174/174 | 174/174 | 174/174 | | 174/174 |
| AbSSR56 | 225/225 | 225/225 | 225/225 | | 219/225 | -/- | 219/219 | | 225/225 |
| AbSSR57 | 257/257 | 257/257 | 257/257 | | 257/257 | 257/257 | 257/257 | | 257/257 |
| AbSSR58 | 165/169 | 167/169 | 169/169 | | 167/169 | 167/167 | 167/169 | | 167/169 |
| AbSSR59 | 197/201 | 197/201 | 197/201 | | 197/201 | 197/201 | 197/201 | | 197/201 |
| AbSSR60 | 206/208 | 206/208 | 206/208 | | 206/208 | 206/208 | 208/208 | | 206/208 |
| AbSSR64 | 104/104 | 104/104 | 104/104 | | 104/104 | 104/104 | 104/104 | | 104/104 |
| AbSSR65 | 197/213 | 188/213 | 213/213 | | 188/213 | 188/213 | 188/213 | | 188/213 |

Table 3 gathers and summarises the genotypic differences between the tested strains.

| | | A15 | LB811 | LB906 | J10117 | J10165 | J11500 | J9277 |
|---|---|---|---|---|---|---|---|---|
| **LB811** | Nb of differences<br>Nb of markers | 4<br>22 | | | | | | |
| **LB906** | Nb of differences<br>Nb of markers | 5<br>21 | 5<br>22 | | | | | |
| **J10117** | Nb of differences<br>Nb of markers | 5<br>22 | 4<br>22 | 5<br>21 | | | | |
| **J10165** | Nb of differences<br>Nb of markers | 3<br>22 | 7<br>22 | 5<br>21 | 7<br>22 | | | |
| **J11500** | Nb of differences<br>Nb of markers | 5<br>20 | 8<br>20 | 7<br>19 | 7<br>20 | 4<br>20 | | |
| **J9277** | Nb of differences<br>Nb of markers | 5<br>22 | 7<br>22 | 7<br>21 | 9<br>22 | 5<br>22 | 8<br>20 | |

With this panel of 22 SSR markers, each of the strains shows a unique genotype profile. The number of different alleles between the strains (pair comparisons) is presented in the table above.

Each of the strains presents at least one rare or unique allele:

- 2 for LB811: AbSSR58 (165), AbSSSR65 (197)
- 1 for LB906: AbSSR02 (192)
- 1 for J10117: AbSSR12 (174)
- 1 for J10165: AbSSR56 (219)
- 3 for J11500: AbSSR12 (173), AbSSR14 (165), AbSSR42 (156)
- 2 for J9277 : AbSSR45 (197), AbSSR57 (219)

The hybrids of the invention may also be distinguished from other mushroom strains by analysing the different alleles at the *MAT* locus. *A. bisporus* has a multi-allelic unifactorial system of sexual inter-compatibility under control of the *MAT* locus having 14 alleles (Xu et al., 1993, Imbernon et al., 1995). Due to the sexual reproduction and life cycle of *A. bisporus,* the genotype of fertile fruiting heterokaryons is always heterozygous at the *MAT* locus and therefore heteroallelic at the markers linked to the *MAT* locus, such as the P1N150 marker.

The P1N150 genotype of the hybrid LB811 is a "4/7" heterokaryotic genotype profile.
The P1N150 genotype of the hybrid LB906 is a "3/4" heterokaryotic genotype profile.
The P1N150 genotype of the hybrid J10117 is a "2/4" heterokaryotic genotype profile.
The hybrids of the invention may be unambiguously identified by analysing these SSRs or MAT locus markers. Any suitable PCR primers that bracket the defined marker regions may be used for this purpose. Methods of designing and using suitable PCR primers are well-known in the art. Sequencing methods may also be used with this respect.

***Derivatives of the hybrids of the invention***

The present invention also encompasses cells "deriving" from the above-mentioned hybrids. These cells are hereafter called **"derivatives",** wherein said derivatives are *Agaricus bisporus* hybrids comprising all the SSR molecular markers and/or P1N150 allelic sequences of the hybrid **LB811** having been deposited under the CNCM Accession Number I-4945 or of the hybrid **LB906** having been deposited under the CNCM Accession Number I-4946 or of the hybrid **J10117** having been deposited under the CNCM Accession Number 1-4950,

wherein the P1N150 allelic combinations for LB811, LB906 and J10117 are the ones as in Table 17,

and wherein the SSR molecular markers for LB811, LB906 and J10117 are the ones as in Table 15bis.

Methods of obtaining said derivatives include: somatic selection, tissue culture selection, single spore germination, multiple spore germination, mating between mycelium obtained from homokaryotic or heterokaryotic spores from these hybrids, selfing, repeated mating back to the initial culture, mutagenesis, trait conversion, introgressive trait conversion, inbreeding and transformation, to provide some examples. As used herein **transformation** is the process of introducing new genetic material into the genome of the initial culture by biotechnological means (Velcko et al., 2004). **Introgressive trait conversion** herein represents the mating of a line of interest with a strain such that a desired trait from the strain of interest is introduced into a predominating genetic background of the other strain. **Trait conversion** represents the selective introduction of the genetic determinants of one or more desirable traits into the genetic background of an initial strain while retaining most of the genetic background of the initial strain. Percentages of the initial genotype that will be present in *A. bisporus* derivatives range from almost 100% in the case of somatic selections, to 99.x% in the case of strains modified by DNA-mediated transformation, to 90-99.x% in the case of single or multiple spore selections or some mutagenesis, to an average of approximately 75% to approximately 85% in the case of sibling-offspring matings (= selfing). Many methods of genotype determination are known in the art to determine the percentage of DNA of an initial culture that is present in another culture.
The present description discloses cultures substantially benefiting from the use of a line obtained from strains LB811 or LB906 in their development, such as hybrid offspring (or progeny) having a line obtained from strain LB811 or LB906 as a parent.
These **descendent** hybrids or lines (also called **"offspring"** or **"progeny")** obtained by using the hybrids of the invention are encompassed by the present invention and can be designated as "derivatives". They can be homokaryons or heterokaryons.
Usually, **descendent** hybrids are obtained by growing two homokaryons side by side on a single petri dish, allowing their growth (incubator) until a contact is established between them. For a sexually compatible cross, subsequently a dense ('stroma') or fast-growing mycelium will develop at the interface. It can be picked, transferred to new plates and grown (incubator).
In another aspect, the present description also discloses a process of producing a new hybrid mushroom culture, comprising mating the homokaryon obtained from the hybrids LB811 or LB906 or J10117 with a second *A. bisporus* homokaryon.
In another aspect, the present invention furthermore relates to a process of producing a new hybrid mushroom culture, comprising mating the homokaryon obtained from the hybrids LB811 or LB906 or J10117 with a second *A. bisporus* heterokaryon.
In another aspect, the present description also discloses a process of producing a new hybrid mushroom culture, comprising mating the hybrids LB811, LB906 or J10117 with a second *A. bisporus* homokaryon.
The heterokaryotic hybrids or homokaryotic lines obtained by these processes are encompassed within the invention, as "hybrid derivatives".
The scope of the present invention also encompasses the hybrids or lines obtained from strain LB811 or LB906 or J10117, having a genetic trait introduced through introgressive matings of offspring back to the line obtained from strains LB811, LB906, J10117 or through transformation.
In this embodiment derivatives of hybrids are unambiguously recognisable by their genotype, which will be predominantly a subset of that of the single initial culture (including the 22 SSR markers and the P1N150 marker disclosed above).

Being derived from the same homokaryotic line, the hybrids LB811 and LB906 share common molecular markers and/or sequences with the homokaryon **J10117s62** (see Table 4 below).

Table 4 presents the SSR markers of the hybrids of the invention and of the homokaryon J10117s62.

| SSR marker | LB811 | LB906 | J10117s62 |
|---|---|---|---|
| AbSSR02 | 188/188 | 188/192 | 188 |
| AbSSR05 | 329/332 | 329/332 | 332 |
| AbSSR12 | 171/171 | 171/171 | 171 |
| AbSSR13 | 212/212 | 212/212 | 212 |
| AbSSR14 | 167/167 | 167/167 | 167 |
| AbSSR19 | 191/191 | 191/191 | 191 |
| AbSSR23 | 176/176 | 176/176 | 176 |
| AbSSR31 | 167/167 | 167/167 | 167 |
| AbSSR33 | 192/192 | 192/192 | 192 |
| AbSSR36 | 149/157 | 149/149 | 149 |
| AbSSR39 | 186/188 | 186/188 | 188 |
| AbSSR42 | 177/179 | 179/179 | 179 |
| AbSSR43 | 231/231 | nd | nd |
| AbSSR45 | 193/203 | 193/203 | 203 |
| AbSSR49 | 174/174 | 174/174 | 174 |
| AbSSR56 | 225/225 | 225/225 | 225 |
| AbSSR57 | 257/257 | 257/257 | 257 |
| AbSSR58 | 165/169 | 167/169 | 169 |
| AbSSR59 | 197/201 | 197/201 | 201 |
| AbSSR60 | 206/208 | 206/208 | 206 |
| AbSSR64 | 104/104 | 104/104 | 104 |
| AbSSR65 | 197/213 | 188/213 | 213 |

Moreover, the homokaryotic parent J10117s62 has the allelic marker P1N150 "4" at the *MAT* locus.

The present description also discloses *Agaricus bisporus* hybrids comprising (on at least one allele per locus) all the SSR molecular markers and/or P1N150 allelic sequence of the homokaryon **J10117s62.**

In another embodiment the description also discloses *Agaricus bisporus* hybrids comprising all the SSR molecular markers and/or P1N150 allelic sequences of the hybrid **LB811** having been deposited under the CNCM Accession Number I-4945 or of the hybrid **LB906** having been deposited under the CNCM Accession Number I-4946 or of the hybrid **J10117** having been deposited under the CNCM Accession Number I-4950.

The said hybrids advantageously have a smooth white colour cap, an improved canning yield, improved agronomic value and an appropriate phenotype for producer and consumer satisfaction.

More precisely, an embodiment of this invention is an *A. bisporus* heterokaryon comprising, on one of its allele per locus, the same SSR markers as a line obtained from the hybrids LB811, LB906 or J10117 for at least 50% of the markers listed on Table I above. In other embodiments, the present invention encompasses heterokaryons comprising on one of its alleles per locus the same SSR marker as a line obtained from the hybrids LB811, LB906 or J10117 for at least 55%, 60%, 65%, 70%, 75%, 80%, 85%, 90%, 95%, 96%, 97%, 98%, 99% or essentially 100% of the markers listed on Table I above. In a preferred embodiment, said *A. bisporus* heterokaryon additionally has the P1N150 allelic sequence "4" (for J10117-, LB811- and LB906-derived cells) or "7" (for LB811-derived cells) or "3" (for LB906-derived cells) or "2" (for J10117-derived cells).

***Cell cultures and products incorporating the hybrids of the invention***

In another aspect, the present invention relates to a hybrid mushroom culture of *Agaricus bisporus* comprising any of the *Agaricus bisporus* hybrids of the invention, as described above. Importantly this hybrid mushroom culture exhibits an improved canning yield, an improved agronomic value (agronomic yield, mushroom weight and size, etc.) and an appropriate phenotype for producer and consumer satisfaction (lightness, smoothness, stipe diameter, etc.). In particular, it is vegetatively incompatible with white commercial mushrooms strains.

In particular, the present invention relates to hybrid mushroom culture of *Agaricus bisporus* comprising:

- the *Agaricus bisporus* hybrid **LB811** which has been deposited with the Collection Nationale de Cultures de Microorganismes (CNCM) of the Institut Pasteur, 25, rue du Docteur Roux, F-75724 PARIS CEDEX 15, FRANCE on January 20, 2015, by SOMYCEL and whose CNCM Accession No. is **I-4945,** or derivatives thereof, or

- the *Agaricus bisporus* hybrid **LB906** which has been deposited with the Collection Nationale de Cultures de Microorganismes (CNCM) of the Institut Pasteur, 25, rue du Docteur Roux, F-75724 PARIS CEDEX 15, FRANCE on January 20, 2015, by SOMYCEL and whose CNCM Accession No. is **I-4946,** or derivatives thereof, or

- the *Agaricus bisporus* hybrid **J10117** which has been deposited with the Collection Nationale de Cultures de Microorganismes (CNCM) of the Institut Pasteur, 25, rue du Docteur Roux, F-75724 PARIS CEDEX 15, FRANCE on January 20, 2015, by SOMYCEL and whose CNCM Accession No. is **I-4950** or derivatives thereof.

In another aspect the present invention relates to any product incorporating said hybrid mushroom culture of the invention. In a preferred embodiment said product is selected from the group consisting of: mycelium, spawn, inoculum, casing inoculum and colonised substrates (e.g. grain, compost, casing and friable particulate matter).

These products may be obtained as indicated above, by conventional means.

In another aspect the description also discloses any part of these cultures. Said part may be selected from the group consisting of hyphae, spores and parts of cells, said parts including e.g. nuclei, mitochondria, cytoplasm, protoplasts, DNA, RNA, proteins, cell membranes and cell walls, each part being present in either the vegetative mycelium of the culture or in mushrooms produced by the culture or both.

In a preferred embodiment the present invention relates to any cell of the *Agaricus bisporus* cultures described above.

In another preferred embodiment, the present invention relates to a spore comprising a cell of the *Agaricus bisporus* cultures described above.

More generally the present description also discloses culture or product incorporating the SSR molecular markers and/or P1N150 allelic sequences of lines obtained from the hybrids of the invention. In a preferred embodiment said culture or product comprises the same alleles as an initial line obtained from LB811, LB906 or J10117 for at least 70%, 80%, 85%, 90%, 95%, 96%, 97%, 98%, 99% or essentially 100% of the SSR markers or P1N150 allelic sequences.

***Homokaryons of the hybrids of the invention***

Homokaryons are specific derivatives of the hybrids of the invention; they may be obtained by any conventional means. Isolating single spore isolates (SSI) under appropriate conditions is an example: spores of the hybrids of the invention can be diluted in liquid medium and plated on a nutritive agar medium. Alternate methods of de-heterokaryotisation (e.g. protoplasting mycelium or cells) can also be used.

Several methods have been described in the scientific literature to identify and select homokaryons: phenotypic methods (slow mycelium growth, different aspect of mycelium), specific abilities (fruiting ability, crossing ability), genotypic methods (enzymatic markers, molecular markers, sequencing techniques ...) or different combinations.

In a specific aspect the present invention relates to homokaryons (or homokaryotic lines) obtained from the hybrids of the invention or from derivatives thereof.

In particular the present invention relates to homokaryons (or homokaryotic lines) obtained from:

- The *Agaricus bisporus* hybrid **LB811** which has been deposited with the Collection Nationale de Cultures de Microorganismes (CNCM) of the Institut Pasteur, 25, rue du Docteur Roux, F-75724 PARIS CEDEX 15, FRANCE on January 20, 2015 by SOMYCEL and whose CNCM Accession No. is **I-4945,** or from derivatives thereof, or from

- The *Agaricus bisporus* hybrid **LB906** which has been deposited with the Collection Nationale de Cultures de Microorganismes (CNCM) of the Institut Pasteur, 25, rue du Docteur Roux, F-75724 PARIS CEDEX 15, FRANCE on January 20, 2015 by SOMYCEL, whose CNCM Accession No. is **I-4946,** or from derivatives thereof, or from

- The *Agaricus bisporus* hybrid **J10117** which has been deposited with the Collection Nationale de Cultures de Microorganismes (CNCM) of the Institut Pasteur, 25, rue du Docteur Roux, F-75724 PARIS CEDEX 15, FRANCE on January 20, 2015 by SOMYCEL and whose CNCM Accession No. is **I-4950** or from derivatives thereof.

More particularly the present description also discloses the *Agaricus bisporus* homokaryon **J10117s62** which has been deposited with the Collection Nationale de Cultures de Microorganismes (CNCM), Institut Pasteur, 25 rue du Docteur Roux, 75724 PARIS Cedex 15 on January 20, 2015 by SOMYCEL under the CNCM Accession No. **I-4948.** The deposit will be maintained in the depository for a period of 30 years, or 5 years after the last request, or for the effective life of the patent, whichever is longer and will be replaced as necessary during that period.
Is also disclosed by the present description an *A. bisporus* homokaryon comprising the same SSR markers as a line obtained from the hybrids LB811, LB906 or J10117 for at least 70%, 80%, 85%, 90%, 95%, 96%, 97%, 98%, 99% or essentially 100% of the markers listed on Table I above. In a preferred embodiment, said *A. bisporus* homokaryon additionally has the P1N150 allelic of "4" (for J10117-, LB811- and LB906-derived cells) or "7" (for LB811-derived cells) or "3" (for LB906-derived cells) or "2" (for J10117-derived cells).
The present invention also encompasses cultures incorporating the above-mentioned homokaryons.

***Production of mushrooms from the hybrids of the invention***

The description also discloses a mushroom produced by growing a crop of mushrooms from any of the cultures described above. Mushroom production processes have been detailed above. They are well-known in the art.
In a further aspect the present invention relates to a method of producing hybrid mushrooms, said method comprising:

a) Inoculating a mushroom growth medium with a hybrid *Agaricus bisporus* mushroom hybrid of the invention, namely LB811, LB906 or J10117,

b) Maintaining said inoculated growth medium under conditions conducive to mushroom fruiting and

c) Collecting mushrooms from said growth medium.

***Uses of the hybrids of the invention***

Uses of the culture of mushroom strain LB811, LB906 or J10117 include, among many other things, the production of homokaryotic lines and other offspring from spores or protoplasts of LB811, LB906 or J10117, the production of hybrid mushroom cultures incorporating lines obtained from strain LB811, LB906 or J10117, the production of mushrooms from cultures incorporating lines obtained from LB811, LB906 or J10117 and the production of mushroom parts from cultures incorporating lines obtained from strain LB811, LB906 or J10117.
Still other uses include processes for producing a mushroom culture that comprise mating homokaryotic *Agaricus bisporus* lines obtained from strain LB811, LB906 or J10117 or the heterokaryotic strains LB811, LB906 or J10117 themselves with another mushroom line. Also processes for producing a mushroom culture containing in its genetic material one or more traits introgressed into lines obtained from LB811, LB906 or J10117 through introgressive trait conversion or transformation and to the mushroom cultures, mushrooms and mushroom parts produced by such introgression.
Further the invention may include a hybrid mushroom culture, mushroom, mushroom part, including a spore or culture part produced by mating a homokaryotic line obtained from strain LB811, LB906 or J10117 or an introgressed trait conversion of a line obtained from strain LB811, LB906 or J10117 with another mushroom line.
Still other uses of the present invention include the production of homokaryotic mushroom lines derived from mushroom lines obtained from strain LB811, LB906 or J10117, as well as the processes for making other homokaryotic mushroom lines derived from mushroom lines obtained from strain LB811, LB906 or J10117 and to the production of the inbred mushroom lines and their parts derived by the use of those processes.
The present description also discloses an *A. bisporus* strain which produces mushrooms with improved canning yield, wherein said improved canning yield is controlled by a genetic determinant, wherein said improved canning yield of the *Agaricus bisporus strain* is defined as a normalised canning yield of the said *A. bisporus* strain $NCY_{strain}$ above 100 and wherein the normalised canning yield $NCY_{strain}$ is calculated as follows :

$$NCY_{strain} = CY_{strain} - CY_{control} + 100$$

wherein the CYstrain and $CY_{control}$ correspond respectively to the canning yield of the *A. bisporus* strain and to the canning yield of an *A. bisporus* control strain that does not contain the said genetic determinant and

wherein the canning yield is measured with mushrooms collected at the same harvesting stage according to the procedure as detailed in example 2.

In one embodiment, the normalised canning yield of the strain $NCY_{strain}$ is comprised between 100.1 and 120, particularly between 100.5 and 120, more particularly between 101 and 115, more particularly between 102 and 115, even more particularly between 103 and 110, for example around 110.

The *Agaricus bisporus* strain according to the invention is obtainable by crossing an *A. bisporus* strain J10117 as first parent with a second *A. bisporus* strain as a second parent.

In the *Agaricus bisporus* strain of the invention, the trait responsible for increased canning yield is controlled by a genetic determinant which is inheritable and can be found in J10117.

In one embodiment, the *A. bisporus* strain according to the invention and as described herein before contains an "increased canning yield" trait obtainable from an *A. bisporus* strain J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4950 or LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4945, or LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

In one embodiment of the invention, the "increased canning yield" trait or a strain comprising said trait is obtainable from any of the deposited strains by growing the progeny of said strain. In particular, the "increased canning yield" trait or a strain comprising said trait is obtainable from any of the deposited strains by i) growing propagating material of said strain and growing a mature mushroom therefrom; ii) harvesting the fertile propagating material therefrom, and iii) growing mushrooms from the cultures grown from the propagating material harvested under ii) and selecting strains which grow mushrooms with increased canning yield.

In one embodiment, the description also discloses material obtainable from a mushroom strain according to the invention and as described herein before including, but without being limited thereto mycelium, spawn, spores, protoplasts, herein defined as "propagating material", or any other part or product of the strain which still exhibits the increased canning yield phenotype according to the invention, particularly when grown into a mushroom.

The invention further relates to a method of producing an *A. bisporus* strain producing mushrooms with improved canning yield comprising the steps of

i) providing propagating material of an hybrid *A. bisporus* strain according to the present invention;

ii) growing said material and recovering a reproducing material therefrom;

iii) crossing the reproducing material obtained in ii) with the reproducing material of an *A. bisporus* strain which does not have the trait of increased canning yield, growing propagating material obtained from the cross and harvesting propagating material there from and

iv) growing mushrooms from the propagating material harvested under iii) and selecting a mushrooms strain producing mushrooms with improved canning yield.

In one embodiment, the propagating material, such as spores, used in said method according to the invention is from an *A. bisporus* strain selected from the group consisting of J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

In one embodiment, the *A. bisporus* strain which does not have the trait of improved canning yield which is crossed in step iii) can be any *A. bisporus* strain variety.

Accordingly it is possible to convert any *A. bisporus* strain into an *A. bisporus* strain according to the present invention by crossing of such any *A. bisporus* strain with an *A. bisporus* strain according to the present invention, i.e. that contains the genetic determinant responsible for the improved canning yield trait.

The genetic determinant for the trait of the present invention is obtainable from an *A. bisporus* strain selected from the group consisting of
J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4950 ; LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

Such genetic determinant is inheritable and can be transmitted through generations by techniques known to man skilled in the art of plant/mushroom breeding or plant biotechnology.

An exemplary embodiment of such a method comprises the transfer by introgression of the genetic determinant responsible for increased canning yield, or nucleic acid sequence containing such genetic determinant, from a donor *A. bisporus* strain into a recipient *A. bisporus* strain by crossing the strains.

This transfer can thus suitably be accomplished by using traditional breeding techniques. Genetic determinant is thus introgressed in some embodiments into commercial *Agaricus* strain using marker-assisted selection (MAS) or marker-assisted breeding (MAB). MAS and MAB involve the use of one or more of the molecular markers for the identification and selection of those offspring strains that contain the genetic determinant that is associated with the desired trait, i.e. improved canning yield. In the context of the presently disclosed subject matter, such identification and selection is based on selection of genetic determinant of the presently disclosed subject matter or markers associated therewith.

*A. bisporus* strains developed according to these embodiments can advantageously derive a majority of their traits from the recipient strain, and derive improved canning yield trait from the donor strain. As discussed herein above, traditional breeding techniques can be used to introgress a genetic determinant responsible for improved canning yield into a recipient *A. bisporus* strain. In some embodiments, a donor *A. bisporus* strain that exhibits improved canning yield and comprises a genetic determinant encoding for improved canning yield is crossed with a recipient *A. bisporus* strain that in some embodiments exhibits commercially desirable characteristics.

In a method of recurrent selection and backcrossing, improved canning yield trait, i.e. the genetic determinant for the trait, can be introgressed into a target recipient strain (the recurrent parent) by crossing the recurrent parent with a first donor strain, which differs from the recurrent parent and is referred to herein as the "non-recurrent parent". The recurrent parent is a strain that does not have improved canning yield but does possess commercially desirable characteristics. The donor strain may advantageously be the *A. bisporus* strain selected in the group consisting of J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

The description also discloses the use of a genetic determinant, to confer improved canning yield, particularly at the harvesting stage, to an *A. bisporus* strain lacking said genetic determinant, wherein said genetic determinant is obtainable from *A. bisporus* strain selected in the group consisting of J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

The man skilled in the art can easily implement the invention in any *A. bisporus* strain and the information and tool herein provided allow this implementation of the invention. The deposited *A. bisporus* strains do contain the genetic determinant of the present invention. Any of the deposited strains can be used by the man skilled in the art as a source for the genetic determinant, associated with improved canning yield, particularly at harvesting stage.

The progeny resulting from a cross between the recurrent parent and non-recurrent parent can be backcrossed to the recurrent parent. The resulting population can then be screened for improved canning yield.

Following screening, the strains that exhibit an improved canning yield phenotype or, in some embodiments, genotype and thus comprise the requisite genetic determinant, responsible for increased canning yield, are then selected and backcrossed to the recurrent parent for a number of generations in order to allow for the *A. bisporus* strain to become increasingly inbred. As used herein, the terms "introgression", "introgressed" and "introgressing" refer to the process whereby one gene or a plurality of genes, a QTL or haplotype of one species, variety or strain are moved into the genome of another species, variety or strain, by crossing those species. The crossing may be natural or artificial. The process may optionally be completed by backcrossing to the recurrent parent, in which case introgression refers to infiltration of the genes of one species into the gene pool of another through repeated backcrossing of an interspecific hybrid with one of its parents. An introgression may also be described as a heterologous genetic material stably integrated in the genome of a recipient strain.

As used herein, the expression "trait" refers to a characteristic or phenotype, e.g., mushroom cap colour or a disease or pathogen resistance. A trait may be inherited in a dominant or recessive manner, or in a partial or incomplete-dominant manner. A trait may be monogenic (i.e. determined by a single locus) or polygenic (i.e. determined by more than one locus) or may also result from the mutual interaction among genes or interaction of one or more genes with the environment.

As used herein, the expression "phenotype" or "phenotypic trait" refers to the appearance or other detectable characteristic of an individual, resulting from the interaction of its genome, proteome and/or metabolome with the environment.

"Genetic determinant" is understood within the scope of the invention to mean a gene or a locus in the genome or part thereof that is capable of contributing to the canning yield of the mushrooms of the strain by influencing expression trait at the level of the DNA itself, at the level of translation, transcription and/or activation of a final polypeptide product, i.e., to regulate metabolism in mushroom tissue leading to the phenotypic expression of the genotype. A genetic determinant is inheritable, i.e. it can be transferred to progeny by crossing.

"Donor *A. bisporus* strain" is understood within the scope of the invention to mean the *A. bisporus* strain which provides the genetic determinant associated to significantly improved canning yield.

"Improvement in canning yield" and "improved canning yield" are understood within the scope of the invention to mean a mushroom which exhibits a higher canning yield as calculated in Example 2, which is statistically significant at $P < 0.05$ or $P < 0.01$ compared to mushroom from a control strain, particularly a control strain A15 which is a commercial *A. bisporus* freely available and that can be purchased or any other strain regardless of its origin (commercial, collection, wild ...).

The expression "harvesting stage" corresponds to the maturity stage of the mushroom at which the mushroom is sufficiently developed for being harvested , i.e. the cap is sufficiently independent from the stem and the size corresponds to the usual commercial grade.

The expression "propagating material" refers to any mushroom material that is suitable for growing and fructification, but not crossing, and includes spores, mycelium, spawn and protoplasts for example. This material is heterokaryotic.

The expression "reproducing material" relates to any mushroom material that is suitable and capable of being crossed, but not capable of fructification. This material is homokaryotic and corresponds to homokaryons, ie homokaryotic mycelium.

Accordingly, based on the description of the present invention, the skilled person in possession of *a strain selected in the group consisting of* J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4946 has no difficulty transferring the trait of improved canning yield of the present invention, i.e. the genetic determinant directing such a trait, to any other *A. bisporus* strain of various types using breeding techniques well-known in the art. The trait of the present invention is for example transferred to *A. bisporus* strains producing mushrooms of various types or shapes, exhibiting different levels of agronomic yield.

In one embodiment, the genetic information determining the trait of improved canning yield, particularly at harvesting stage, according to the instant invention comprises a genetic determinant that is on at least one locus that is/are obtainable from *A. bisporus* strain *selected in the group consisting of* J10117 deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4950, LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945 and LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4946.

The description also discloses a new trait in *A. bisporus* mushroom that allows a better yield in the processing of harvested mushroom for the canning industry. The genetic determinant supporting this trait while not disclosed in terms of DNA sequence does however exist and is inheritable. This genetic determinant contained in the deposited material is the tool to bestow the desired trait of improved canning yield as defined above into any *A. bisporus* strain that lacks this trait. This trait can easily be measured and quantified using the testing procedure as depicted in Example 2 herein disclosed.

The deposits have been made in order to provide the skilled person with a source of improved canning yield trait that can be used for reproducing the claimed invention and there is no need to repeat any breeding program to develop strains with the trait since this trait is present in the deposited strains and is easily transferable to any *A. bisporus* strain.

## Figure legends

**Figure 1** represents the evolution of cumulative yield (kg mushrooms / $m^2$) across time (days after casing) in two small scale trials (each trial being represented on A or B) for LB811, LB906 and control strains A15 and Sylvan 512.

**Figure 2** represents the evolution of cumulative yield (kg mushrooms / $m^2$) across time (days after casing) in two medium scale trials. The experimental strains and the control were grown simultaneously in the same room. A: for three batches of LB906 (as compared with A15). B: for three batches of LB811 (as compared with A15).

**Figure 3** represents the evolution of cumulative yield (kg mushrooms / $m^2$) across time (days after casing) in two large scale trials. The experimental strains and the control were grown simultaneously in separate parallel rooms. A: for LB811 and LB906 (as compared with A15). B: for two batches of LB906 (as compared with A15).

## Examples

### *1. Production of the hybrids of the invention*

1.1. *Material*

**[0157]** * **LB906** is a hybrid obtained from a cross plan involving the homokaryon J10117s62 and the homokaryon 76s14.

A deposit of a culture of the *Agaricus bisporus* homokaryon **J10117s62** has been made with the Collection Nationale de Cultures de Microorganismes (CNCM), Institut Pasteur, 25 rue du Docteur Roux, 75724 PARIS Cedex 15. The date of deposit was January 20, 2015. Its CNCM Accession No. is **I-4948.**

A deposit of a culture of the *Agaricus bisporus* homokaryon **76s14** has been made with the Collection Nationale de Cultures de Microorganismes (CNCM), Institut Pasteur, 25 rue du Docteur Roux, 75724 PARIS Cedex 15. The date of deposit was January 20, 2015. Its CNCM Accession No. is **I-4947.**

**[0160]** * **LB811** is a hybrid obtained from a cross plan involving the homokaryon J10117s62 and the homokaryon FS9-18-s50 issued from the heterokaryon FS9-18.

The homokaryon J10117s62 has been described above.

A deposit of a culture of the *Agaricus bisporus* heterokaryon **FS9-18** has been made with the Collection Nationale de Cultures de Microorganismes (CNCM), Institut Pasteur, 25 rue du Docteur Roux, 75724 PARIS Cedex 15. The date of deposit was January 20, 2015. Its CNCM Accession No. is **I-4949.**

1.2. *Mating of the homokaryotic lines*

Crosses can be performed on plates. Each parental homokaryon is grown on an agar plate (PDA, compost-agar ...). A plug from each parental homokaryon is transferred to a fresh plate so that the two homokaryons are inoculated side by side, separated by a few centimetres. The plate is incubated so that the two homokaryons grow until they meet.

At the contact point, if they are sexually compatible, a new mycelium starts growing, i.e. the hybrid. It can be picked and transferred to a new fresh plate and incubated.

1.3. *Lab culture of the hybrid strains of the invention*

Our hybrids are grown on compost-agar plates (adjusted at pH 7) and transferred using a punch as agar plugs from plate to plate. Plates are grown in an incubator at 24 °C until they colonise one to two thirds of the plate and then transferred to the cool room at 2 °C.

All microbiology operations are performed under sterile conditions (laminar flow hood, sterile instruments, media and packages).

***2. Evaluation of the properties of the hybrids of the invention***

2.1. *Canning yield (CY)*

CY can be assessed by processing mushrooms on a commercial production line, a pilot production line or R&D lab devices and equipment replicating a commercial process, whilst measuring the weight at each step of the process. By this way one can determine the drained weight and calculate the ratio of the weight of drained canned mushrooms for ex vs. weight of fresh mushrooms introduced at the beginning of the process. Thus the total CY can be obtained by combining the evacuation %, cooling %, blanching % and sterilization %.
Appropriate experimental design and statistical analysis are used in order to compare different treatments.
When these conditions are met, two or more varieties (e.g. hybrids developed from a breeding programme) can be compared to each other or to a control (e.g. commercial strain).
For all the canning yield trials, the process was as follows:

- Reception of the mushrooms,
- Determination of weight per box,
- Evacuation and hydration (negative pressure to ensure entrance of free water in the mushrooms),
- Determination of weight per box,
- Storage at cold store,
- Determination of weight per box,
- Blanching (12 min 90 °C),
- Cooling in box (15 °C moving water),
- Draining of free water (2 min),
- Determination of weight per box,
- Cutting into slices (6.25 mm lab slicing machine),
- Filling 580 ml jars with net weight between 330-340 g mushrooms per jar,
- Maximum filling of jars with brine,
- Closing of jars,
- Sterilisation of jars (mass autoclave),
- Storage (min 5 days),
- Opening of jars,
- Draining (2 min),
- Weighing.

All batches were randomised with regards to processing order.
Data of the experimental strains were normalized considering that the CY value of the control was 100%, according to the following formula:

$$NCY_{strain} = CY_{strain} - CY_{control} + 100$$

whereby:

NCY: Normalized Canning Yield
CY: Canning Yield

Sylvan A15 was the control in each trial.

a) The canning yield of LB811 and LB906 has been assessed in 2 trials.

In the first trial, mushrooms were sorted according to grade, small (< 45 mm) or large (> 45 mm) and the effect of the size of the mushrooms studied. The plan was to harvest all mushrooms on the same day for a delivery to the canning plant under codes, however LB906 was faster and decision was taken to harvest on 2 consecutive days and to compare as two different treatments.
Table 5 and 5bis summarise the results obtained on two independent trials:

Table 5: Results of statistical analysis on canning yield data (analysis of variance and comparison of means).

| Code | Strain name | Origin | Picking date | NCY (%) | * | Average (%) |
|---|---|---|---|---|---|---|
| 1 | **A15** | Sylvan commercial strain | D | 100.00 | B | 100.00 |
| 2 | **LB811** | Sylvan Exp. hybrid | D | 109.67 | A | 109.87 |
| 3 | **LB906** | Sylvan Exp. hybrid | D-1 | 110.19 | A | |
| 4 | **LB906** | Sylvan Exp. hybrid | D | 109.74 | A | |
| *Data followed by the same letter are not statistically different (Tukey test α = 5%) | | | | | | |

Table 5bis: Results of statistical analysis on canning yield data (analysis of variance and comparison of means).

| Code | Strain name | Origin | Picking date | NCY (%) | * | Average (%) |
|---|---|---|---|---|---|---|
| 1 | **A15** | Sylvan Commercial strain | D | 100.00 | B | 99.98 |
| 4 | **512** | Sylvan Commercial strain | D | 99.95 | B | |
| 2 | **LB811** | Sylvan Exp. hybrid | D | 105.82 | A | 107.05 |
| 3 | **LB906** | Sylvan Exp. hybrid | D | 108.28 | A | |
| *Data followed by the same letter are not statistically different (Tukey test α = 5%) | | | | | | |

The CY values of the control A15 were comparable with those of previous blank trials.

For both trials, the statistical analysis showed a very highly significant strain effect and a very significant size effect (larger mushrooms producing a higher CY). For LB906 the effect of the duration of storage (mushrooms processed on day D or D + 1) was not significant.

In the first trial (table 3), the results did not show significant differences among the group of the experimental hybrids (average 109.87%). However a significant difference was shown compared to the commercial control (100.0 %) with almost 9.9 % higher values for the experimental hybrids, which is a substantial difference for the canning industry (as suggested by Coale et al., 1972).

In the second trial (table 3bis), the mushrooms were processed regardless of their size and therefore the effect of the size of mushrooms was not studied. The results did not show significant differences within the group of commercial strains (average 99.98%) or within the experimental hybrids (average 107.05%). However the differences between experimental and control strains were significant with almost 7.1% higher values for the experimental strains in average compared to the controls.

9.9% and 7.1% higher values of canning yield are substantial differences for the processing industry (as suggested by Coale et al., 1972).

b) The CY of the parent strain J10117 has furthermore been assessed in similar conditions.

Table 6 shows the results obtained by comparing the CY of J10117 and commercial strains Sylvan A15 and Sylvan 512.

The analysis performed for all 3 trials confirmed the statistically higher CY of J10117, as compared with A15 and 512 strains (see table 6).

| Code | Strain name | Origin | Normalised Canning Yield (%) | | | | | | | | | | | |
|---|---|---|---|---|---|---|---|---|---|---|---|---|---|---|
| | | | Trial 1 | | | | Trial 2 | | | | Trial 3 | | | |
| | | | Flush 1 | * | Flush 2 | * | Flush 1 | * | Flush 2 | * | Flush 1 | * | Flush 2 | * |
| A | **J10117** | Exp. hybrid | 108,35 | A | 101,79 | A | 102,16 | AB | 109,37 | A | 109,43 | A | 108,09 | A |
| B | **A15** | Comm. Strain | **100,00** | B | **100,00** | B | **100,00** | BC | **100,00** | B | **100,00** | C | **100,00** | BC |
| C | **512** | Comm. Strain | 98,93 | B | 99,50 | B | 102,01 | AB | 98,25 | B | 107,25 | B | 99,70 | C |

| Strain name | Origin | | Canning yield data (%) | | | |
|---|---|---|---|---|---|---|
| | | | **Three trials (mean)** | | | |
| | | | **Flush 1** | * | **Flush 2** | * |
| **J10117** | Sylvan | Exp. hybrid | **106.58** | **A** | **106.41** | **A** |
| **A15** | Sylvan | Comm. strain | **100.00** | **D** | **100.00** | **B** |
| **512** | Sylvan | Comm. strain | **103.06** | **BC** | **99.15** | **B** |

Table 6: Normalized Canning yield data (%) and results of statistical analysis

Treatments followed by the same letter were not significantly different.

For LB906, a further evaluation was performed at larger scale. The growing trials consisted of cultivating LB906 and a control strain (512) in a growing facility at the same time, in different rooms in parallel.

Mushrooms were mechanically harvested at the same harvesting stage and delivered to a mushroom canning facility on the day of harvest.

Upon reception, the mushrooms were hydrated in vacuum and cool stored until processing.

When more than 3 tons of fresh mushrooms of the same quality from each strain were delivered, they were separated and processed as batches in cans, to determine the processing yield for each strain on a canning production line. The process was similar as described previously.

CY of a batch was calculated as the ratio of the weight of mushrooms in cans divided by the weight of fresh mushrooms. The weight of mushrooms in cans was calculated by multiplying the average drained weight of a number of cans sampled from the batch (13 to 20 cans per batch) by the number of cans produced in the same batch.

To avoid a bias, the CY data was considered for statistical analysis (T Student test) only when the storage time applied to the batch was in the normal range for the factory. 9 and 10 series of data were considered for LB906 and 512, respectively. The NCY was calculated as indicated previously.

LB906 expressed an average NCY of 103.0 vs. 100.0 for the control, i.e. a higher value by 3.0 %, and the difference was statistically significant (Table *).

Therefore, the significant advantage of LB906 for the CY was confirmed.

Table 6bis : Normalised canning yield (NCY) data (%) and results of statistical analysis.

| Strain name | Normalised Canning Yield data | Average difference | T Student test analysis Conclusion |
|---|---|---|---|
| 512 | 100.0 ± 3.2 % | 3.0% | 0.04 (< 0.05) significant |
| LB906 | 103.0 ± 3.5 % | | |

2.2. *Vegetative incompatibility*

Adding mycelium to the casing (as cac'ing, i.e. colonised compost or CI, i.e. Casing Inoculant, a proprietary material) is commonly used in commercial mushroom production to improve earliness and evenness of the first flush. This practice can therefore be used to test for vegetative incompatibility.

Practically strain A is spawned in the compost and its mycelium colonises the compost (spawn run). At the end of spawn run strain B is mixed into the casing layer, the mix is laid on the surface of the compost and its mycelium colonises the casing layer (case run). In a normal growing schedule the mycelium from the compost and the mycelium from the casing will anastomose at the interface of the two layers, forming a singular fungal network throughout the substrate. Pins of the first flush will form, grow and develop into mushrooms. The development of mycelium, pins and mushrooms on the surface can be assessed (aspect and timing) to determine if the two strains are vegetatively compatible.

If two strains A and B are vegetatively compatible, the mycelium will anastomose, pinning will occur and mushrooms will appear and develop in the usual and expected time frame.

Whereas if the two strains A and B are vegetatively incompatible, the mycelium of the two strains will not anastomose, pinning will not occur and mushrooms will not appear in the usual and expected time frame. The antagonism will prevent or delay the appearance and development of the mushrooms.

Romaine & Royse (2011), using genetic transformation, have established that after anastomosis of two compatible strains the mushrooms of the first flush will be of the strain B genotype (grown in the casing), whereas mushrooms of second flush will be of the strain A genotype (spawned in the compost).

Tested strains were grown in trays (1.4 $m^2$) filled with compost spawned with strain A (so-called base spawn). The tray was covered with a plastic sheet pierced with large rings (15.2 cm diameter, 3.8 cm deep) thus creating individual plots. Each compatibility test was replicated in 5 or 10 of the individual plots.

At the end of the spawn-run casing was mixed with CI (Casing Inoculant) of strain B at a rate of 375 $g/m^2$ and applied on the ring plots in order to test the compatibility of the A/B combination.

All combinations of A/B strains were tested for compatibility with A in the compost / B in the casing and reciprocally B in the compost / A in the casing. The A/A and B/B combinations were used as controls.

All test plots were watered on the day of application then grown using the parameters of commercial mushroom production.

Visual observations were made daily including, strength of mycelium growth, pin production and timing of any crop. Ratings of the casing surface at day 8 (growth of mycelium) and day 16 (presence of pins and mushrooms) are summarised in the following table.

If no fructification occurs for the A/B combination or if only pins appear with no further development or a delay compared to A/A or B/B,, A and B were deemed to be vegetatively incompatible.

Table 7 shows the results obtained for the strains of the invention, as compared with control A15 and parental strains.

Table 7: Vegetative compatibility of different *Agaricus bisporus* strains

| | | Ratings | Strain in the compost | | | | | |
|---|---|---|---|---|---|---|---|---|
| | | **Conclusion** | **A15** | **J10117** | **76** | **FS9.18** | **LB811** | **LB906** |
| **Strain in the casing** | **A15** | Day 8 | Very good growth | No growth | No growth | No growth | No growth | No growth |
| | | Day 16 | Mushrooms | No mushrooms | No mushrooms | No mushrooms | No mushrooms | No mushrooms |
| | | **Conclusion** | **Compatible** | **Not compatible** | **Not compatible** | **Not compatible** | **Not compatible** | **Not compatible** |
| | **J10117** | Day 8 | No growth | Very good growth | nd | nd | No growth | Little surface growth |
| | | Day 16 | No mushrooms | Mushrooms | | | No mushrooms | No mushrooms |
| | | **Conclusion** | **Not compatible** | **Compatible** | | | **Not compatible** | **Not compatible** |
| | **76** | Day 8 | nd | No growth | Very good growth | No growth | nd | No growth |
| | | Day 16 | | No mushrooms | Mushrooms | No mushrooms | | No mushrooms |
| | | **Conclusion** | | **Not compatible** | **Compatible** | **Not compatible** | | **Not compatible** |
| | **FS9.18** | Day 8 | No growth | nd | No growth | Very good growth | No growth | nd |
| | | Day 16 | No mushrooms | | No mushrooms | Mushrooms | No mushrooms | |
| | | **Conclusion** | **Not compatible** | | **Not compatible** | **Compatible** | **Not compatible** | |
| | **LB811** | Day 8 | No growth | No growth | No growth | No growth | Very good growth | No growth |
| | | Day 16 | No mushrooms | No mushrooms | No mushrooms | No mushrooms | Mushrooms | No mushrooms |
| | | **Conclusion** | **Not compatible** | **Not compatible** | **Not compatible** | **Not compatible** | **Compatible** | **Not compatible** |
| | **LB906** | Day 8 | Good growth | No growth | No growth | No growth | No growth | Very good growth |
| | | Day 16 | No mushrooms | No mushrooms | No mushrooms | No mushrooms | No mushrooms | Mushrooms |
| | | **Conclusion** | **Not compatible** | **Not compatible** | **Not compatible** | **Not compatible** | **Not compatible** | **Compatible** |

Table 7bis shows the results obtained for the strains of the invention, as compared with control commercial strains (U1and A15 and derivatives thereof 512, 520, 737), parental strains and other strains (J9277, J10165, J11500).

| | Ratings Conclusion | Strain in the compost | | | | | | | | | | | | |
|---|---|---|---|---|---|---|---|---|---|---|---|---|---|---|
| | | **U1** | **A15** | **512** | **520** | **737** | **J10117** | **76** | **FS9.18** | **LB811** | **LB906** | **J9277** | **J10165** | **J11500** |
| **U1** | Day 9 | Very good growth | Very good growth | Good growth | Very good growth | Very good growth | No growth | No growth | No growth | No growth | No growth | No growth | No growth | No growth |
| | Day 16 | Mush-rooms | Mush-rooms | Mush-rooms | Mush-rooms | Mush-rooms | No mush-rooms | No mush-rooms | No mush-rooms | No mush-rooms | No mush-rooms | No mush-rooms | No mush-rooms | No mush-rooms |
| | **Conclusion** | **Compatible** | **Compatible** | **Compatible** | **Compatible** | **Compatible** | **Not compatible** | **Not compatible** | **Not compatible** | **Not compatible** | **Not compatible** | **Not compatible** | **Not compatible** | **Not compatible** |
| **A15** | Day 9 | Very good growth | Very good growth | Very good growth | Very good growth | Very good growth | No growth | No growth | No growth | No growth | No growth | No growth | No growth | No growth |
| | Day 16 | Mush-rooms | Mush-rooms | Mush-rooms | Mush-rooms | Mush-rooms | No mush-rooms | No mush-rooms | No mush-rooms | No mush-rooms | No mush-rooms | No mush-rooms | No mush-rooms | No mush-rooms |
| | **Conclusion** | **Compatible** | **Compatible** | **Compatible** | **Compatible** | **Compatible** | **Not compatible** | **Not compatible** | **Not compatible** | **Not compatible** | **Not compatible** | **Not compatible** | **Not compatible** | **Not compatible** |
| **512** | Day 9 | Good growth | Very good growth | Very good growth | Very good growth | Very good growth | No growth | No growth | No growth | No growth | No growth | No growth | No growth | No growth |
| | Day 16 | Mush-rooms | Mush-rooms | Mush-rooms | Mush-rooms | Mush-rooms | No mush-rooms | No mush-rooms | No mush-rooms | No mush-rooms | No mush-rooms | No mush-rooms | No mush-rooms | No mush-rooms |
| | **Conclusion** | **Compatible** | **Compatible** | **Compatible** | **Compatible** | **Compatible** | **Not compatible** | **Not compatible** | **Not compatible** | **Not compatible** | **Not compatible** | **Not compatible** | **Not compatible** | **Not compatible** |
| **520** | Day 9 | Very good growth | Very good growth | Very good growth | Very good growth | Very good growth | No growth | No growth | No growth | No growth | No growth | No growth | No growth | No growth |
| | Day 16 | Mush-rooms | Mush-rooms | Mush-rooms | Mush-rooms | Small Mush-rooms | No mush-rooms | No mush-rooms | No mush-rooms | No mush-rooms | No mush-rooms | No mush-rooms | No mush-rooms | No mush-rooms |
| | **Conclusion** | **Compatible** | **Compatible** | **Compatible** | **Compatible** | **Compatible** | **Not compatible** | **Not compatible** | **Not compatible** | **Not compatible** | **Not compatible** | **Not compatible** | **Not compatible** | **Not compatible** |
| **737** | Day 9 | Very good growth | Very good growth | Very good growth | Very good growth | Very good growth | No growth | No growth | No growth | No growth | No growth | No growth | No growth | No growth |
| | Day 16 | Mush-rooms | Mush-rooms | Mush-rooms | Mush-rooms | Mush-rooms | No mush-rooms | No mush-rooms | No mush-rooms | No mush-rooms | No mush-rooms | No mush-rooms | No mush-rooms | No mush-rooms |
| | **Conclusion** | **Compatible** | **Compatible** | **Compatible** | **Compatible** | **Compatible** | **Not compatible** | **Not compatible** | **Not compatible** | **Not compatible** | **Not compatible** | **Not compatible** | **Not compatible** | **Not compatible** |

(continued)

| | Ratings Conclusion | Strain in the compost | | | | | | | | | | | | |
|---|---|---|---|---|---|---|---|---|---|---|---|---|---|---|
| | | **U1** | **A15** | **512** | **520** | **737** | **J10117** | **76** | **FS9.18** | **LB811** | **LB906** | **J9277** | **J10165** | **J11500** |
| **J10 117** | Day 9 | No growth | No growth | No growth | No growth | No growth | Very good growth | Low growth | No growth | No growth | Low growth | No growth | No growth | No growth |
| | Day 16 | No mush-rooms | No mush-rooms | No mush-rooms | No mush-rooms | No mush-rooms | Mush-rooms | No mush-rooms | No mush-rooms | No mush-rooms | No mush-rooms | No mush-rooms | No mush-rooms | No mush-rooms |
| | **Conclu-sio n** | **Not com-patible** | **Not com-patible** | **Not com-patible** | **Not com-patible** | **Not com-patible** | **Compati-ble** | **Not com-patible** | **Not com-patible** | **Not com-patible** | **Not com-patible** | **Not com-patible** | **Not com-patible** | **Not com-patible** |
| **76** | Day 9 | No growth | No growth | No growth | No growth | No growth | No growth | Very good growth | No growth | No growth | No growth | No growth | No growth | No growth |
| | Day 16 | No mush-rooms | No mush-rooms | No mush-rooms | No mush-rooms | No mush-rooms | No mush-rooms | Mush-rooms | No mush-rooms | No mush-rooms | No mush-rooms | No mush-rooms | No mush-rooms | No mush-rooms |
| | **Conclu-sio n** | **Not com-patible** | **Not com-patible** | **Not com-patible** | **Not com-patible** | **Not com-patible** | **Not com-patible** | **Compati-ble** | **Not com-patible** | **Not com-patible** | **Not com-patible** | **Not com-patible** | **Not com-patible** | **Not com-patible** |
| **FS9 . 18** | Day 9 | No growth | No growth | No growth | No growth | No growth | No growth | No growth | Very good growth | No growth | No growth | No growth | No growth | No growth |
| | Day 16 | No mush-rooms | No mush-rooms | No mush-rooms | No mush-rooms | No mush-rooms | No mush-rooms | No mush-rooms | Mush-rooms | No mush-rooms | No mush-rooms | No mush-rooms | No mush-rooms | No mush-rooms |
| | **Conclu-sio n** | **Not com-patible** | **Not com-patible** | **Not com-patible** | **Not com-patible** | **Not com-patible** | **Not com-patible** | **Not com-patible** | **Compati-ble** | **Not com-patible** | **Not com-patible** | **Not com-patible** | **Not com-patible** | **Not com-patible** |
| **LB8 11** | Day 9 | No growth | No growth | No growth | No growth | No growth | No growth | No growth | No growth | Very good growth | No growth | No growth | No growth | No growth |
| | Day 16 | No mush-rooms | No mush-rooms | No mush-rooms | No mush-rooms | No mush-rooms | No mush-rooms | No mush-rooms | No mush-rooms | Mush-rooms | No mush-rooms | No mush-rooms | No mush-rooms | No mush-rooms |
| | **Conclu-sio n** | **Not com-patible** | **Not com-patible** | **Not com-patible** | **Not com-patible** | **Not com-patible** | **Not com-patible** | **Not com-patible** | **Not com-patible** | **Compati-ble** | **Not com-patible** | **Not com-patible** | **Not com-patible** | **Not com-patible** |
| **LB9 06** | Day 9 | No growth | Good growth | Low growth | Low growth | Low growth | No growth | No growth | No growth | No growth | Very good growth | No growth | No growth | No growth |
| | Day 16 | No mush-rooms | No mush-rooms | Pins | Pins | Pins | Pins | No mush-rooms | No mush-rooms | No mush-rooms | Mush-rooms | No mush-rooms | No mush-rooms | No mush-rooms |
| | **Conclu-sio n** | **Not com-patible** | **Not com-patible** | **Not com-patible** | **Not com-patible** | **Not com-patible** | **Not com-patible** | **Not com-patible** | **Not com-patible** | **Not com-patible** | **Compati-ble** | **Not com-patible** | **Not com-patible** | **Not com-patible** |

(continued)

| | Ratings Conclusion | Strain in the compost | | | | | | | | | | | | |
|---|---|---|---|---|---|---|---|---|---|---|---|---|---|---|
| | | **U1** | **A15** | **512** | **520** | **737** | **J10117** | **76** | **FS9.18** | **LB811** | **LB906** | **J9277** | **J10165** | **J11500** |
| **J9277** | Day 9 | No growth | No growth | No growth | No growth | No growth | No growth | No growth | Low growth | No growth | No growth | Very good growth | No growth | No growth |
| | Day 16 | No mushrooms | No mushrooms | No mushrooms | No mushrooms | No mushrooms | No mushrooms | No mushrooms | Pins | No mushrooms | No mushrooms | Mushrooms | No mushrooms | No mushrooms |
| | **Conclusion** | **Not compatible** | **Not compatible** | **Not compatible** | **Not compatible** | **Not compatible** | **Not compatible** | **Not compatible** | **Not Compatible** | **Not compatible** | **Not compatible** | **Compatible** | **Not compatible** | **Not compatible** |
| **J10165** | Day 9 | No growth | No growth | No growth | No growth | No growth | No growth | No growth | Low growth | No growth | No growth | No growth | Very good growth | No growth |
| | Day 16 | No mushrooms | No mushrooms | No mushrooms | No mushrooms | No mushrooms | No mushrooms | No mushrooms | Pins | No mushrooms | No mushrooms | No mushrooms | Mushrooms | No mushrooms |
| | **Conclusion** | **Not compatible** | **Not compatible** | **Not compatible** | **Not compatible** | **Not compatible** | **Not compatible** | **Not compatible** | **Not Compatible** | **Not compatible** | **Not compatible** | **Not compatible** | **Compatible** | **Not compatible** |
| **J11500** | Day 9 | No growth | No growth | No growth | No growth | No growth | No growth | No growth | Low growth | No growth | No growth | No growth | No growth | Very good growth |
| | Day 16 | No mushrooms | No mushrooms | No mushrooms | No mushrooms | No mushrooms | No mushrooms | No mushrooms | Pins | No mushrooms | No mushrooms | No mushrooms | No mushrooms | Mushrooms |
| | **Conclusion** | **Not compatible** | **Not compatible** | **Not compatible** | **Not compatible** | **Not compatible** | **Not compatible** | **Not compatible** | **Not Compatible** | **Not compatible** | **Not compatible** | **Not compatible** | **Not compatible** | **Compatible** |

Table 7bis : Vegetative compatibility of different *Agaricus bisporus* strains.

It is well known that all current commercial white strains are derived from U1 and are therefore vegetatively compatible with each other (Romaine & Royse, 2011). In particular, the commercial white strain A15 is compatible with Sylvan 512,Sylvan 520 and Sylvan 737.

Of note, Somycel 76 and Fungisem 9.18 are not derived from U1.

However, the hybrids of the invention are not compatible with any of the tested strains and, in particular, with the commercial white strain A15.

Moreover, J10117 is not compatible with the commercial white strain A15.

The hybrids of the invention can therefore be considered as "virus breaking" strains.

### 2.3. Agronomic results

2.3.1. *Cap colour and surface aspect*

Measurements have been performed on a Minolta chromameter according to the L,a,b colour opponent space with dimension L for lightness and a & b for the colour-opponent dimensions.

Parameters: 30 mushrooms/strain, 1 measure (top of the cap)/mushroom, for flushes 1 & 2, peak of the flush (before picking).

In the L,a,b system, the colour is described by 3 components: L (lightness, 0 = black to 100 = white), a (- green to red +), b (- blue to yellow +).

Analysis of variance (F test) & comparison of means (Tukey test $\alpha = 5\%$) for the 3 variables have been performed.

The surface aspect (5 rates: very smooth, smooth, medium scaly, scaly, very scaly) and the visual appearance i.e. colour perception (5 rates: very white, white, off-white, cream) of the mushrooms produced by the hybrids of the invention have been assessed.

The mushrooms produced by LB811 and LB906 have a visually white pileus colour.

LB811 and LB906 exhibited higher values of L (lightness) in the first flush, even significantly higher for LB906. In the second flush, L values were comparable to the controls for LB811 but significantly, slightly lower for LB906.

Beside the cap colour *stricto sensu,* the smoothness of the cap also generates the impression of brighter and/or whiter mushrooms compared to current commercial strains such as Sylvan A15 grown in the same trials. The appearance or visual aspect is more important than the absolute colour values as for consumers it is the key to trigger the act of purchasing on the fresh market.

Strain 'U1' has inherited an intermediate phenotype between its smooth-white and off-white (scaly) parents (Fristche, 1981). It is slightly scaly to medium scaly in the first flush and smoother from the second flush. All derivatives of U1 express a similar phenotype (e.g. A15 and 512).

By contrast LB811 and LB906 have been consistently smoother than the A15 control grown in the same conditions in first, second and third flushes, regardless of the scale (small to large) and location (France, England, Belgium) of the trials.

Table 8 shows the surface colour and visual aspects of the mushrooms produced by the hybrids of the invention, as compared with control 512 and A15 strains.

**Table 8:** Comparison of 3 colour parameters and 2 visual ratings for flushes 1 & 2, in trials 1 & 2 (controls A15 & S12).

| Parameter Trial | Colour measurements parameters | | | | | | | | | | | | Visual ratings | | | |
|---|---|---|---|---|---|---|---|---|---|---|---|---|---|---|---|---|
| | L | | | | a | | | | b | | | | Surface aspect | | Visual appearance | |
| | 1 | | 2 | | 1 | | 2 | | 1 | | 2 | | 1 | 2 | 1 | 2 |
| **Flush 1** | | | | | | | | | | | | | | | | |
| 512 | 92.04 | C | 92.38 | D | -0.65 | BC | -0.50 | A | 8.03 | C | 6.83 | A | Smooth | Smooth | White | White |
| A15 | 91.97 | C | 92.65 | CD | -0.57 | B | -0.57 | AB | 8.90 | A | 7.31 | AB | Medium scaly | Medium scaly | White | White |
| LB811 | **93.81** | **A** | 93.10 | ABC | -0.75 | C | -0.63 | BC | 8.51 | ABC | 8.25 | CD | Very smooth | Very smooth | Very white | Very white |
| LB906 | **93.36** | **AB** | **93.66** | **A** | -0.28 | A | -0.73 | D | 8.21 | BC | 8.13 | BC | Very smooth | Very smooth | Very white | Very white |
| **Flush 2** | | | | | | | | | | | | | | | | |
| 512 | 93.83 | ABC | 93.62 | A | -0.75 | C | -0.69 | BC | 8.08 | CD | 7.50 | CD | Smooth | Smooth | White | White |
| A15 | 93.40 | A | 93.94 | A | -0.68 | BC | -0.59 | B | 7.96 | D | 7.22 | D | Smooth | Smooth | White | White |
| LB811 | 93.50 | C | 94.29 | A | -0.75 | C | -0.84 | C | 8.24 | BCD | 8.00 | BC | Very smooth | Very smooth | Very white | Very white |
| LB906 | 92.12 | D | 90.61 | B | -0.13 | A | 0.26 | A | 8.78 | AB | 9.16 | A | Very smooth | Very smooth | Very white | Very white |

*2.3.2. Phenotypic characterisation*

a) Methods:

Morphology of the mushrooms has been measured by means of a Mitutoyo electronic calliper, with the following parameters: 100 mushrooms/strain, 4 measures, for flushes 1 & 2, peak of flush (after picking).
Cap diameter (CapD), cap height (CapH), stipe diameter (StipeD) and stipe height (StipeH) have been measured.
The following ratios have been calculated for statistical analysis:

$$\text{Ratio 1} = \text{CapD} / \text{CapH}$$

$$\text{Ratio 2} = \text{StipeH} / \text{StipeD}$$

The variance has been calculated (F test) and the means have been compared (Tukey test = 5%) for the 6 variables.

b) Results:

The morphology of the hybrids LB906 and LB811 are characterised on Table 9.

Table 9: Cap/stipe diameter and cap/stipe height of the hybrids of the invention Numbers followed by the same letter are not significantly different.

| **Parameter** | Cap Diameter (mm) | | | | Cap Height (mm) | | | | **Ratio 1** | | | | Stipe Diameter (mm) | | | | Stipe Height (mm) | | | | **Ratio 2** | | | |
|---|---|---|---|---|---|---|---|---|---|---|---|---|---|---|---|---|---|---|---|---|---|---|---|---|
| **Trial** | 1 | | 2 | | 1 | | 2 | | 1 | | 2 | | 1 | | 2 | | 1 | | 2 | | 1 | | 2 | |
| **Flush 1** | | | | | | | | | | | | | | | | | | | | | | | | |
| 512 | 30.02 | A | 32.38 | C | 21.48 | ABC | 20.29 | C | 1.58 | C | 1.60 | D | 15.55 | A | 14.29 | D | 28.22 | C | 31.96 | AB | 1.83 | C | 2.26 | A |
| A15 | 34.45 | A | 35.39 | AB | 20.92 | BC | 21.53 | AB | 1.65 | B | 1.64 | C | 16.18 | A | 16.15 | AB | 27.61 | C | 29.95 | B | 1.71 | C | 1.87 | C |
| LB811 | 34.09 | A | 34.48 | B | 22.08 | A | 21.47 | AB | 1.54 | C | 1.60 | CD | 14.39 | B | 15.19 | C | 32.30 | A | 33.62 | A | 2.26 | A | 2.26 | A |
| LB906 | 32.90 | A | 36.99 | A | 19.52 | D | 20.75 | BC | 1.68 | AB | 1.78 | A | 15.56 | A | 16.65 | A | 31.32 | AB | 33.59 | A | 2.02 | B | 2.03 | BC |
| **Flush 2** | | | | | | | | | | | | | | | | | | | | | | | | |
| 512 | 27.26 | D | 33.20 | BC | 17.06 | C | 18.99 | B | 1.60 | C | 1.75 | B | 13.79 | B | 15.72 | A | 28.26 | BC | 31.69 | B | 2.06 | BC | 2.03 | B |
| A15 | 30.60 | C | 32.34 | C | 18.28 | B | 18.46 | BC | 1.68 | B | 1.75 | B | 13.56 | B | 15.80 | A | 31.13 | A | 28.67 | C | 2.33 | A | 1.82 | C |
| LB811 | 33.60 | A | 35.63 | A | 20.79 | A | 20.94 | A | 1.61 | C | 1.70 | BC | 15.49 | A | 14.95 | BC | 24.92 | D | 32.07 | B | 1.62 | E | 2.16 | B |
| LB906 | 32.18 | ABC | 33.20 | BC | 18.06 | B | 17.87 | C | 1.78 | A | 1.86 | A | 14.88 | A | 14.57 | BC | 30.89 | A | 34.97 | A | 2.09 | B | 2.43 | A |

LB811 is characterized by a narrow straight stipe and a rounded cap.

More specifically, stipe diameter was significantly and repeatedly lower compared to the controls, characterising a narrow stipe.

LB906 is characterised by a straight stipe and a less rounded cap compared to commercial controls.

More specifically, ratio 1 (= cap diameter / cap height) was significantly and repeatedly lower compared to the controls, characterising an oblate cap.

Thus, the hybrids of the invention (J10117, LB906, and LB811) have an appropriate phenotype for mechanical harvesting (straight stipe, mushrooms growing individually with even distribution on the crop) and a good overall quality of mushrooms.

2.3.3. *Agronomic trials*

a) Methods

Trials have been performed at small, medium and large scale in different facilities. Experimental designs were created to control the vertical climatic gradient for each growing room and plots were randomised within each level. The number of plots and plot size increased from the earliest to the latest steps of screening.

For small scale trials, plots were 0.12 $m^2$ tubs (2 to 15 plots split across 5 levels), filling rate 75.5 kg compost/$m^2$, spawning rate 0.7 %, spawn run 12 days in tubs (phase II), casing 53% blonde peat - 21% black peat - 9% limestone - 17% gypsum, case run 9 days and ruffling 2 days before airing.

For medium scale trials, plots were 1.45 $m^2$ trays (3 to 12 plots split across 4 levels), filling rate 83 kg compost/$m^2$, spawning rate 0.5 %, spawn-run 15 days in trays (phase II), casing 80% black peat -10% blonde peat -10% sugar beet lime, cacing 375 g/$m^2$ and case-run 8 days.

For large scale trials, spawn-run was 16 days in a bulk tunnel (phase III), spawning rate 0.5%. trays 1.5 $m^2$ (20 or 25 plots/strain, split across 5 levels) were filled at 90 kg incubated compost/$m^2$, supplemented at 1.33 kg/$m^2$ (MC Substradd), cased with 100% peat, cacing 300 g/$m^2$ and case-run 7 days.

A commercial control was included in each trial (Sylvan A15). In the early steps of the screening (small, medium and large scale trials), the new strains and control were grown in the same room (mixed rooms). In the later steps (medium and large scale trials), the control was grown simultaneously in an identical and parallel room (separate rooms) with the cultural practice adapted to each strain in order to favour their growth and production and assess their optimised potential.

Watering was adapted to the requirements of each treatment and plot.

Mushrooms were picked at stage 2-3 for each plot and treatment and weight was recorded daily.

In small scale trials, together with yield, number of mushrooms per plot was also recorded daily.

Earliness (timing) was assessed both from the date of the first pick (picking of the first mushrooms) and from the date of the peak of the first flush (day when the maximum weight of mushrooms were picked) by comparing the data of the strain to the data of the commercial control.

Yield potential was assessed as the weight of mushrooms per surface unit (i.e. kg/$m^2$) from trials performed at small, medium and large scale, compared to 1 or 2 commercial controls (Sylvan A15, Sylvan 512) with appropriate experimental designs including internal replications.

Components of yield (number of mushrooms and average weight of mushrooms = weight / number) were assessed from small scale trials.

b) Results

An improved timing is an advantage in terms of crop management, mushroom quality and economically, as the beginning of the cropping period is cleaner from pests and diseases, compared to later days of the first flush and later flushes of the crop.

Yet, the current near-global monoculture for *A. bisporus* restricts the range of cultural characteristics available to mushroom growers (Romaine & Royse, 2011) and therefore distinct features such as timing and distribution of yield across time during cropping may be seen as advantages in the current context.

Table 10 shows the timing of LB811 and LB906 in small, medium and large scale trials (expressed by comparison with the commercial control Sylvan A15).

Compared to the commercial control, LB906 showed a better timing in terms of first pick in 66% of the tests and in terms of peak of the first flush in 64% of the tests. Therefore LB906 could be described as an early strain compared to the control.

Compared to the commercial control, LB811 showed better timing in terms of first pick in 48% of the tests and in terms of peak of the first flush in 60% of the tests. Therefore LB811 could be described as an early strain compared to the control, though to a lesser extent compared to LB906.

Figures 1 to 3 present the cumulative yield across time for the hybrids of the invention as compared to commercial controls (A15, Sylvan 512) in small, medium or large scale trials.

As observed on figures 1 (A and B), 2A and 3 (A and B), LB906's timing is always better as compared to the commercial strains A15 and Sylvan 512. Moreover, LB906's timing is further improved compared to LB811 on small scale (see figure 1A and 1B), and on large scale (see figure 3A).

As far as LB811 is concerned, figures 2B and 3A show that its timing is better than the control strains on medium scale and on large scale, although it is not better on small scale trials (figures 1A and 1B).

The enclosed figures therefore show the improved timing (i.e. earlier pick of mushrooms in the first flush), higher contribution of the first days of the first flush to the total crop yield, and yield potential of the hybrids of the invention compared to the commercial controls.

Table 10: timing of LB811 and LB906 in small, medium and large scale trials

| Strain | Scale | Type (mixed / separate rooms) | Nb of trials | Nb of tests | **Date of first pick of 1st flush**<br>**Timing* of control - timing* of new strain (days)**<br>**(in the same trial)** | | | | | | | **Date of peak of 1st flush**<br>**Timing* of control - timing* of new strain (days)**<br>**(in the same trial)** | | | | | | |
|---|---|---|---|---|---|---|---|---|---|---|---|---|---|---|---|---|---|---|
| | | | | | **-2** | **-1** | **0** | **1** | **2** | **3** | **4** | **-2** | **-1** | **0** | **1** | **2** | **3** | **4** |
| | | | | | | | | | | | | | | | | | | |
| LB811 | Small | Mixed | 12 | 31 | 1 | 0 | 11 | 8 | 9 | 2 | 0 | 0 | 0 | 14 | 17 | 0 | 0 | 0 |
| LB811 | Medium | Mixed | 13 | 44 | 0 | 0 | 26 | 4 | 14 | 0 | 0 | 0 | 4 | 12 | 21 | 6 | 1 | 0 |
| LB811 | Medium | Separate | 0 | / | / | / | / | / | / | / | / | / | / | / | / | / | / | / |
| LB811 | Large | Mixed | | 2 | 0 | 0 | 1 | 0 | 1 | 0 | 0 | 0 | 0 | 0 | 1 | 0 | 1 | 0 |
| LB811 | Large | Separate | | 3 | 0 | 0 | 3 | 0 | 0 | 0 | 0 | 0 | 0 | 2 | 1 | 0 | 0 | 0 |
| | | | | **80** | **1** | **0** | **41** | **12** | **24** | **2** | **0** | **0** | **4** | **28** | **40** | **6** | **2** | **0** |
| | | | | | ***1 (1.3%)*** | | ***41*** *(51.3%)* | ***38 (47.5%)*** | | | | ***4 (5.0%)*** | | ***28 (35.0%)*** | ***48 (60.0%)*** | | | |
| | | | | | **Timing* of control -timing* of new strain (days)**<br>**(in the same trial)** | | | | | | | **Timing* of control - timing* of new strain (days)**<br>**(in the same trial)** | | | | | | |
| **-2** | **-1** | **0** | **1** | **2** | **3** | **4** | **-2** | **-1** | **0** | **1** | **2** | **3** | **4** | | | | | |
| | | | | | | | | | | | | | | | | | | |
| LB906 | Small | Mixed | 13 | 27 | 0 | 0 | 7 | 1 | 9 | 8 | 2 | 0 | 0 | 6 | 21 | 0 | 0 | 0 |
| LB906 | Medium | Mixed | 11 | 38 | 1 | 0 | 14 | 2 | 21 | 0 | 0 | 0 | 2 | 14 | 16 | 0 | 6 | 0 |
| LB906 | Medium | Separate | 1 | 6 | 0 | 0 | 3 | 0 | 3 | 0 | 0 | 0 | 0 | 2 | 1 | 0 | 3 | 0 |
| LB906 | Large | Mixed | | 2 | 0 | 0 | 1 | 1 | 0 | 0 | 0 | 0 | | 1 | 0 | 1 | 0 | 0 |
| LB906 | Large | Separate | | 3 | 0 | 0 | 0 | 3 | 0 | 0 | 0 | 0 | 0 | 2 | 0 | 1 | 0 | 0 |
| | | | | **76** | **1** | **0** | **25** | **7** | **33** | **8** | **2** | **0** | **2** | **25** | **38** | **2** | **9** | **0** |
| | | | | | ***1 (1.3%)*** | | ***25 (32.9%)*** | ***50 (65.8%)*** | | | | ***2 (2.6%)*** | | ***25 (32.9%)*** | ***49 (64.5%)*** | | | |

Table 11 shows the agronomic yield (kg/$m^2$) of LB811 and LB906 in small scale trials:

| | First flush | | | | Final data (first + second flushes) | | | |
|---|---|---|---|---|---|---|---|---|
| | Trial 1 | | Trial 2 | | Trial 1 | | Trial 2 | |
| 512 (control) | 12.82 | A | 10.03 | C | 22.23 | A | 18.89 | A |
| A15 (control) | 13.48 | A | 10.57 | BC | 21.99 | A | 19.10 | A |
| LB811-1 | 9.94 | B | 8.43 | D | 15.95 | C | 16.13 | B |
| LB811-2 | 10.31 | B | 8.25 | D | 16.21 | C | 15.31 | B |
| LB811-3 | 9.91 | B | 9.79 | C | 15.86 | C | 16.61 | B |
| LB906-1 | 13.46 | A | 12.04 | A | 20.60 | B | 18.75 | A |
| LB906-2 | 13.79 | A | 11.47 | AB | 20.42 | B | 19.12 | A |
| Numbers followed by the same letter are not significantly different (Tukey test, $\alpha$ = 5%). | | | | | | | | |

Table 12 shows the number of mushrooms of LB811 and LB906 (mushrooms / plot) in small scale trials:

| | First flush | | | | Final data (first + second flushes) | | | |
|---|---|---|---|---|---|---|---|---|
| | Trial 1 | | Trial 2 | | Trial 1 | | Trial 2 | |
| 512 (control) | 151.81 | A | 104.00 | A | 312.03 | A | 224.67 | A |
| A15 (control) | 148.33 | A | 100.53 | A | 289.20 | A | 216.93 | A |
| LB811-1 | 109.80 | B | 77.07 | B | 188.80 | C | 170.87 | CD |
| LB811-2 | 107.80 | B | 79.53 | B | 182.60 | C | 160.00 | D |
| LB811-3 | 100.20 | B | 95.27 | AB | 170.93 | C | 168.60 | CD |
| LB906-1 | 147.53 | A | 101.93 | A | 246.80 | B | 183.73 | BC |
| LB906-2 | 157.80 | A | 95.73 | AB | 243.93 | B | 192.13 | B |
| Numbers followed by the same letter are not significantly different (Tukey test, $\alpha$ = 5%). | | | | | | | | |

Further, larger mushrooms can constitute an advantage for the fresh market as hand picking is a major, if not the main, production cost: when mushrooms are larger and heavier, the individual act of picking is more efficient and therefore picking productivity is improved.

The average weight of the mushrooms of the invention was therefore assessed.

Table 13 shows the average weight of mushrooms (g/mushroom) for LB906 and LB811:

| | First flush | | | | Second flush | | | |
|---|---|---|---|---|---|---|---|---|
| | Trial 1 | | Trial 2 | | Trial 1 | | Trial 2 | |
| 512 (control) | 10.13 | C | 11.74 | B | 7.08 | C | 8.83 | C |
| A15 (control) | 10.96 | ABC | 12.68 | B | 7.26 | C | 8.76 | C |
| LB811-1 | 10.83 | ABC | 13.43 | AB | 9.19 | AB | 9.94 | BC |
| LB811-2 | 11.49 | AB | 12.55 | B | 9.55 | AB | 10.64 | AB |
| LB811-3 | 11.84 | A | 12.61 | B | 10.20 | A | 11.31 | A |
| LB906-1 | 11.01 | ABC | 14.61 | A | 8.80 | B | 9.86 | BC |
| LB906-2 | 10.48 | BC | 14.63 | A | 9.36 | AB | 9.46 | BC |
| Numbers followed by the same letter are not significantly different (Tukey test, $\alpha$ = 5%). | | | | | | | | |

In this small scale trial, when LB811 is grown in the same growing room under the same conditions as the commercial control, its yield potential is lower compared to the commercial controls both in the first and second flushes (Table 11), as it produces a lower number of mushrooms (Table 12); however the mushrooms of LB811 tend to be larger as indicated by their average weight values (Table 13). Its picking productivity is thus significantly improved.

When grown together with the commercial control, LB906 exhibits a comparable yield (Table 9), with a higher yield in the first flush. LB906 produces a comparable number of mushrooms in the first flush and less mushrooms in the second flush (Table 10); these mushrooms are larger as shown by the average weight values (Table 11). Its picking productivity is thus significantly improved.

LB811 and LB906 were further assessed in medium and large scale specific trials in order to confirm that yield results comparable to commercial controls can be achieved as was seen in the small scale trials (Table 12, Figures 1 to 3).

Results of medium and large scale trials for LB811 show that its final yield can be comparable to commercial controls with a better timing in first flush (Table 12, Figures 2 to 3).

Results for LB906 show that its final yield can be comparable to commercial controls with a better timing in first flush (Table 14, Figures 2 to 3).

Table 14: Agronomic yield (kg/$m^2$) on small, medium and large scale trials for LB811, LB906 and A15 (see next page)

| Scale | First flush | | | Second flush | | | Total | | |
|---|---|---|---|---|---|---|---|---|---|
| | LB811 | LB906 | A15 | LB811 | LB906 | A15 | LB811 | LB906 | A15 |
| Small | 9.97 - 10.34 -9.89 | 13.82-13.44 | 13.44 | 6.04 - 5.89 - 5.96 | 6.64-7.17 | 8.53 | 15.95 -16.21 - 15.86 | 20.42 - 20.60 | 21.99 |
| Small | 8.46 - 8.23 - 9.82 | 11.48-12.00 | 10.57 | 7.70 - 7.02 - 6.80 | 7.63 - 6.72 | 8.53 | 16.13 -15.31 - 16.61 | 19.12 - 18.75 | 19.10 |
| Medium | nd | 10.64-9.57 -10.19 | 12.22 | nd | 9.05 - 9.69 - 8.22 | 7.89 | nd | 19.87 - 19.26 -18.47 | 20.10 |
| Medium | 14.17 - 14.20 - 11.94 | nd | 10.21 | 7.57 - 8.68 - 10.15 | nd | 12.72 | 21.74 - 22.88 -22.09 | nd | 22.92 |
| Large | 15.48 | 16.48 | 14.64 | 9.82 | 10.24 | 11.74 | 29.18 | 29.50 | 30.35 |
| Large | nd | 17.78 | 17.24 | nd | 10.41 | 11.64 | nd | 32.07 | 33.69 |

*2.4. Genotyping*

2.4.1. SSR fingerprinting

a) Methods

12 strains were genotyped by SSR (Single Sequence Repeat, i.e. microsatellite) markers fingerprinting:

- 3 parental heterokaryons: FS9.18, Somycel 76, Sylvan hybrid J10117.
- 3 parental homokaryons: FS9.18s50, 76s14, J10117s62.
- 2 Somycel hybrids (heterokaryons): LB811, LB906.
- 4 controls (heterokaryons, commercial strains): U1, Sylvan A15, Sylvan 512 and Sylvan 520.

Broth cultures of these strains were prepared from plates, by grinding plugs of agar cultures into Potato Dextrose Broth medium. After an incubation period of one to a few weeks at 24 °C (depending on growth speed), broth cultures were filtered (qualitative creped filter paper with 40 μm particle retention). The mycelium was freeze-dried: frozen (-196 °C liquid nitrogen) and lyophilised until dry.

Total DNA was extracted with a classical CTAB-chloroform-isoamyl alcohol protocol, DNA concentrations were adjusted to 25 ng/μl and stored (-20 °C).

The genotyping was performed using 22 co-dominant microsatellite published markers listed in Table 15 (see Foulongne-Oriol et al. 2009 for primer sequences and amplification conditions). Forward primers were labelled with one of the following fluorescent dyes (6-FAM, PET, VIC and NED) as follows:

- 6-FAM: AbSSSR 12 14 23 36 58 64
- PET: AbSSSR 05 13 39 43 56 57
- VIC: AbSSSR 02 19 33 42 49
- NED: AbSSSR 3145 59 60 65

Capillary electrophoresis and fragment size determination were performed on an ABI 3130 sequencer (Applied Biosystems) using GenScan™ 600 LIZ internal standard (Applied Biosystems) (Foulongne-Oriol et al., 2012).

b) Results:

Tables 15 and 15bis present the fingerprints of 4 commercial controls (U1 and 3 derivatives of U1, namely Sylvan A15, 512 and 520), 2 hybrids (LB811 and LB906), their 3 heterokaryotic parents (J10117, FS9.18 and 76) and their 3 homokaryotic parents (J10117s62, FS9.18s50 and 76s14) for 22 SSR markers.
Fingerprints of U1, A15, 512 and 520 show 100% identical results for 22 markers and thus confirm the statement of lack of genetic diversity among the current commercial strains (Foulongne-Oriol et al., 2011, Sonnenberg et al., 2011).
By contrast, LB811 differs from all U1 derivatives by its genotype at 4 markers out of 22 (18.2%): AbSSR 23 39 58 65.
LB906 differs from all U1 derivatives by its genotype at 5 markers out of 22 (22.7%): AbSSR 02 23 36 39 42.
LB811 and LB906 differ from each other by their genotype at 5 markers out of 22 (22.7%): AbSSR 02 36 42 58 65.
The unique fingerprints of the 3 hybrids LB811, LB906 and J10117 at 22 markers are detailed in Table 15.

Table 15 : Genotype fingerprinting of U1 and 3 commercial U1 derivatives with 22 SSR microsatellite markers.

| Markers | U1 | Sylvan A15 | Sylvan 512 | Sylvan 520 |
|---|---|---|---|---|
| AbSSR02 | 188/188 | 188/188 | 188/188 | 188/188 |
| AbSSR05 | 329/332 | 329/332 | nd | 329/332 |
| AbSSR12 | 171/171 | 171/171 | 171/171 | 171/171 |
| AbSSR13 | 212/212 | 212/212 | 212/212 | 212/212 |
| AbSSR14 | 167/167 | 167/167 | 167/167 | 167/167 |
| AbSSR19 | 191/191 | 191/191 | 191/191 | 191/191 |
| AbSSR23 | 170/176 | 170/176 | 170/176 | 170/176 |
| AbSSR31 | 167/167 | 167/167 | 167/167 | 167/167 |
| AbSSR33 | 192/192 | 192/192 | 192/192 | 192/192 |
| AbSSR36 | 149/157 | 149/157 | 149/157 | 149/157 |
| AbSSR39 | 186/186 | 186/186 | 186/186 | 186/186 |
| AbSSR42 | 177/179 | 177/179 | 177/179 | 177/179 |
| AbSSR43 | 231/231 | 231/231 | 231/231 | 231/231 |
| AbSSR45 | 193/203 | 193/203 | 193/203 | 193/203 |
| AbSSR49 | 174/174 | 174/174 | 174/174 | 174/174 |
| AbSSR56 | 225/225 | 225/225 | 225/225 | 225/225 |
| AbSSR57 | 257/257 | 257/257 | 257/257 | 257/257 |
| AbSSR58 | 167/169 | 167/169 | 167/169 | 167/169 |
| AbSSR59 | 197/201 | 197/201 | 197/201 | 197/201 |
| AbSSR60 | 206/208 | 206/208 | 206/208 | 206/208 |
| AbSSR64 | 104/104 | 104/104 | 104/104 | 104/104 |
| AbSSR65 | 188/213 | 188/213 | 188/213 | 188/213 |

Table 15bis: Genotype fingerprinting of the hybrids of the invention and their parents with 22 SSR microsatellite markers.

| Markers | LB811 | LB906 | J10117 | FS9.18 | 76 | J10117s62 | Fs9.18s50 | 76s14 |
|---|---|---|---|---|---|---|---|---|
| AbSSR02 | 188/188 | 188/192 | 188/188 | 188/192 | 188/192 | 188 | 188 | 192 |
| AbSSR05 | 329/332 | 329/332 | 329/332 | 329/329 | 329/332 | 332 | 329 | 329 |
| AbSSR12 | 171/171 | 171/171 | 171/174 | 171/171 | 171/171 | 171 | 171 | 171 |
| AbSSR13 | 212/212 | 212/212 | 212/212 | nd | 212/212 | 212 | 212 | nd |
| AbSSR14 | 167/167 | 167/167 | 167/167 | 167/167 | 167/167 | 167 | 167 | 167 |
| AbSSR19 | 191/191 | 191/191 | 191/191 | 191/191 | 191/191 | 191 | 191 | 191 |
| AbSSR23 | 176/176 | 176/176 | 176/176 | 176/176 | 170/176 | 176 | 176 | 176 |
| AbSSR31 | 167/167 | 167/167 | 167/167 | 153/167 | 167/167 | 167 | 167 | 167 |
| AbSSR33 | 192/192 | 192/192 | 192/192 | 192/192 | 192/192 | 192 | 192 | 192 |
| AbSSR36 | 149/157 | 149/149 | 149/149 | 149/157 | 149/149 | 149 | 157 | 149 |
| AbSSR39 | 186/188 | 186/188 | 186/188 | 186/186 | 186/186 | 188 | 186 | 186 |
| AbSSR42 | 177/179 | 179/179 | 177/179 | 177/177 | 179/179 | 179 | 177 | 179 |
| AbSSR43 | 231/231 | nd | 231/231 | 210/231 | nd | nd | 231 | nd |
| AbSSR45 | 193/203 | 193/203 | 193/203 | 193/195 | 193/195 | 203 | 193 | 193 |
| AbSSR49 | 174/174 | 174/174 | 174/174 | 174/174 | 174/174 | 174 | 174 | 174 |
| AbSSR56 | 225/225 | 225/225 | 225/225 | 225/225 | 225/225 | 225 | nd | nd |
| AbSSR57 | 257/257 | 257/257 | 257/257 | 252/257 | 257/257 | 257 | 257 | 257 |
| AbSSR58 | 165/169 | 167/169 | 169/169 | 165/165 | 167/167 | 169 | 165 | 167 |
| AbSSR59 | 197/201 | 197/201 | 197/201 | 197/197 | 197/203 | 201 | 197 | 197 |
| AbSSR60 | 206/208 | 206/208 | 206/208 | 208/208 | 208/208 | 206 | 208 | 208 |
| AbSSR64 | 104/104 | 104/104 | 104/104 | 104/104 | 104/111 | 104 | 104 | 104 |
| AbSSR65 | 197/213 | 188/213 | 213/213 | 188/197 | 188/188 | 213 | 197 | 188 |

LB811 and LB906 have one Sylvan proprietary parent (J10117) and one commercial or previously commercial parent (respectively, FS9.18 and 76).

LB906 has one proprietary parent (J10117) and one pre-hybrid off-white parent (76). The genotypic fingerprinting shows a polymorphism between the parents:

- When considering the 2 heterokaryotic parents, 11 markers out of 21 (52.4%) show a polymorphism.
- When considering the 2 homokaryotic parents:
  - 11 markers out of 21 (52.4%) do not show a polymorphism between the homokaryotic parents,
  - 8 markers out of 21 (38.1%) show a polymorphism between the homokaryotic parents: AbSSR 02 05 39 45 58 59 60 65.

These markers can be used to show the pedigree of LB906.

LB811 has one proprietary parent (J10117) and one commercial parent (FS9.18). The genotypic fingerprinting shows a polymorphism between the parents:

- When considering the 2 heterokaryotic parents, 14 markers out of 22 (63.6%) show a polymorphism.
- When considering the 2 homokaryotic parents:
  - 14 markers out of 22 (63.6%) do not show a polymorphism between the homokaryotic parents,

- 9 markers out of 22 (40.9%) show a polymorphism between the homokaryotic parents: AbSSR 05 36 39 42 45 58 59 60 65.

These markers can be used to show the pedigree of LB811.
When considering LB811 compared to its heterokaryotic parents, U1 and all its derivatives (i.e. J10117, FS9.18, U1, A15, 512 and 520), 2 markers can discriminate LB811: AbSSR 58 and 65.

2.4.2. Mating-type alleles and genotype at the P1N150 molecular markers

The P1N150 marker is linked to the *MAT* locus (Xu et al., 1993, Kerrigan et al., 1993). Its sequence is available from the H97 V2.0 reference genome sequence (Morin et al., 2012) published by the U.S. Department of Energy Joint Genome Institute (JGI). The 5' end of this marker begins at position 868615 of Scaffold 1. The allelic sequences of P1N150 alleles 1, 2, 3, 4 and 7 are given on SEQ ID NO: 1 to 5 respectively.
U1 and all its derivatives (including Sylvan A15, 512 and 520) are characterised by a '1/2' heteroallelic genotype profile. 'Off-white' heterokaryons (including Somycel 76) are characterized by a '1/3' heteroallelic genotype profile (Imbernon et al., 1995).
The heterokaryotic parents J10117, FS9.18 and 76 are characterized respectively by '2/4', '2/7' and '1/3' heteroallelic genotype profiles.
The homokaryotic parents J10117s62, FS9.18s50h and 76s14 are characterised respectively by '4', '7' and '3' homoallelic genotype profiles.
The LB811 hybrid is characterised by a distinctive '4/7' heterokaryotic genotype profile.
The LB906 hybrid is characterised by a distinctive '3/4' heterokaryotic genotype profile.
Therefore J10117, LB811 and LB906 can be distinguished both from all U1 derivatives, from their heterokaryotic or homokaryotic parents and from each other and on the basis of their P1N150 genotype.

2.4.3. PCR genotyping

Table 16 summarizes the material and methods used for PCR genotyping of the strains of the invention in comparison with other hybrid strains or homokaryon strains such as J10165 (as disclosed in US20100218294 and US2100154079), J11500 (as disclosed in WO/2015/114612) and J9277 (as disclosed in US20080182321 and US2010154079).

| Mar ker | Forward Primer | Reverse Primer | PCR parameters | | | Alleles |
|---|---|---|---|---|---|---|
| | | | Number of cycles | Anneal temperature | Extens ion time | |
| **P1N150** | 5'- AGGC**RY**CC CATCTTCAS C-3' SEQ ID 6 | 5'- GTTCGACG ACGGACTG C-3' SEQ ID 7 | 35 | 56 °C | 1 min | 1 (or IT) 2 3 4 7 |
| **ITS** | 5'- TCCGTAGG TGAACCTG CGG-3' | 5'- TCCTCCGCT TATTGATAT GC-3' | 35 | 56 °C | 1 min | L1 L2 |

(continued)

| Mar ker | Forward Primer | Reverse Primer | PCR parameters | | | Alleles |
|---|---|---|---|---|---|---|
| | | | Number of cycles | Anneal temperature | Extens ion time | |
| | SEQ ID 8 | SEQ ID 9 | | | | L4 L6 L10 |
| **MFPC-1-ELF** | 5'- AY**T**C**R**CAA MAACATAC CTTCAAC-3' SEQ ID 10 | 5'- CATTCGGC GATTTTCTC A-3' SEQ ID 11 | 35 | 55 °C | 0.5 min | E1 E2 E7 E8 E9 |
| **AN** | 5'- GACGATGC GGGACTGG TGGAT-3' SEQ ID 12 | 5'- GGTCTGGC CTAC**R**GGA GTGTTGT- 3' SEQ ID 13 | 35 | 64 °C | 2 min | N1 N2 N5 N6 N7 |
| **AS** | 5'- CCGCCAGC ACAAGGAA TCAAATG- 3' SEQ ID 14 | 5'- TCAGTCGG CCCTCAAA ACAGTC-3' SEQ ID 15 | 35 | 64 °C | 2 min | SD SH SC SB SG |
| **FF** | 5'- TCGGGTGG TTGCAACT GAAAAG-3' SEQ ID 16 | 5'- TTCCTTTCC GCCTTAATT GTTTCT-3' SEQ ID 17 | 35 | 64 °C | 2 min | FF1 FF2 FF4 FF5 FF9 FF10 |

Nucleotic sequences of the forward and reverse primers are depicted in SEQ ID N° 6 to 17.

Based on this genotyping technology, the results are gathered in the Table 17 below (allele 1 is numbered as 1T in this Table in respect of marker P1N150 and details for various alleles for this marker are detailed above). The various alleles for markers ITS, MFPC-1-ELF, AN, AS, FF differ by their sequence polymorphism.

Table 17. Allelic combinations for various tested strains for different markers

| **marker** | **J1011 7** | **J10117 -s62** | **FSfs9fs99 -18** | **FS9 -18-s50** | **LB811** | **LB906** | **A-15** | **J9277** | **J1016 5** | **J1150 0** |
|---|---|---|---|---|---|---|---|---|---|---|
| **P1N15 0** | 2/4 | 4 | 2/7 | 7 | 4/7 | 3/4 | 1T/2 | 1T/4 | 1/2 | 1T/2 |
| **ITS** | I2/I6 | I2/I6 | I10/I10 | I10 | I2/I10 | I1/I2 | I1/I2 | I2/I4 | I2/I5 | I1/I4 |
| **MFPC-1-ELF** | E1/E8 | E1 | E7/E9 | E7 | E1/E7 | E1/E1 | E1/E2 | E1/E2 | E1/E1 | E1/E1 |
| **AN** | N1/N7 | N1 | N6/N6 | N6 | N1/N 6 | N1/N 1 | N1/N 2 | N1/N 5 | N1/N2 | N1/N5 |
| **AS** | SD/SH | SH | SB/SC | SB | nd | SD/SH | SC/SD | SD/SG | SC/SG | SC/SD |
| **FF** | FF2/FF4 | FF4 | FF9/FF10 | FF9 | FF4/FF9 | FF2/FF4 | FF1/FF2 | FF2/FF2 | FF2/FF5 | FF1/FF1 |

This results show again that the strains according to the present invention are genetically different from any known strain.

Table 18 shows the number of genetic difference between the strains based on the tested PCR markers.

| | Nb of markers | A15 | LB811 | LB906 | J10117 | J10165 | J11500 | J9277 |
|---|---|---|---|---|---|---|---|---|
| LB811 | 5 | 5 | | | | | | |
| LB906 | 6 | 5 | 5 | | | | | |
| J10117 | 6 | 6 | 5 | 4 | | | | |
| J10165 | 6 | 5 | 5 | 4 | 6 | | | |
| J11500 | 6 | 4 | 5 | 4 | 6 | 5 | | |
| J9277 | 6 | 5 | 5 | 4 | 6 | 6 | 5 | |

With this panel of 6 PCR markers, each of the strains shows a unique genotype profile.

The number of different alleles between the strains (pair comparisons) is presented in the table 18 above.

**List of references**

Beelman R.B., et al., 2008. Bioactive components in Agaricus bisporus of nutritional, medicinal or biological importance. Mushroom Science, XVII, 1-16.

Callac P., et al., 1993. Morphological, genetic, and interfertility analyses reveal a novel, tetrasporic variety of Agaricus bisporus from the Sonoran Desert of California. Mycologia, 85: 835-851.

Callac P., et al., 2003. A novel homothallic variety of Agaricus bisporus comprises rare tetrasporic isolates from Europe. Mycologia 95:222-231.

Chen S. & Kanaya N., 2012. Protective effects of Agaricus bisporus against cancer and metabolic diseases. Mushroom Science XVIII, 28-33.

Coale C.W.J. & Butz W.T., 1972. Impact of selected economic variables on the profitability of commercial mushroom processing operations. Mushroom Science VIII, 231-237.

Donker H.C.W., et al., 1996. NMR imaging of white button mushrooms (Agaricus bisporus) at various magnetic fields. Magnetic Resonance Imaging 14 (10): 1205-1215.

Fletcher J.T. & Gaze R.H., 2008 Mushroom pests and disease control. A colour handbook. Manson Publishing. ISBN: 978-1-84076-083-4 [Chap. 1. Mushroom growing. p. 7-17; Chap. 2. Mushroom growing systems. p. 18-21; Chap. 6. Viral diseases. p. 109-123]

Foulongne-Oriol M., et al., 2011. Agaricus bisporus cultivars: Hidden diversity beyond apparent uniformity? In: Proceedings of the Seventh International Conference on Mushroom Biology and Mushroom Products. (J.M. Savoie, M. Foulongne-Oriol, M. Largeteau, G. Barroso Eds).Vol. 2. pp 9-16 INRA, France.

Foulongne-Oriol M., et al., 2012. Quantitative trait locus mapping of yield-related components and oligogenic control of the cap color of the button mushroom, Agaricus bisporus. Applied and Environmental Microbiology 78(7): 2422-2434. DOI: 10.1128/AEM.07516-11.

Foulongne-Oriol M., et al., 2009. Novel microsatellite markers suitable for genetic studies in the white button mushroom Agaricus bisporus. Appl. Microbiol. Biotechnol. DOI: 10.1007/s00253-009-2030-8.

Foulongne-Oriol M., et al., 2012. Development of polymorphic microsatellite markers issued from pyrosequencing technology for the medicinal mushroom Agaricus subrufescens. FEMS Microbiol. Lett. 334: 119-126.

Fritsche G., 1983. Breeding Agaricus bisporus at the Mushroom Experimental Station, Horst. The Mushroom Journal, 122, 49-53.

Gao W., et al. 2013 Genetic variation and combining ability analysis of bruising sensitivity in Agaricus bisporus. PloS one, 8 (10). e76826.

Imbernon M., et al., 1995. Allelic polymorphism at the mating-type locus in Agaricus bisporus var. burnettii, and confirmation of the dominance of its tetrasporic trait. Mushroom Science XIV, 1, 11-19.

Kerrigan R.W.K., et al., 1994. The heterothallic life cycle of Agaricus bisporus var. burnettii, and the inheritance of its tetrasporic trait. Exp Mycol 18: 193-210.

Kerrigan R.W.K., et al., 1993. Meiotic behavior and linkage relationships in the secondary homothallic fungus Agaricus bisporus. Genetics, 133: 225-236.

Morin E., et al., 2012. Genome sequence of the button mushroom Agaricus bisporus reveals mechanisms governing adaptation to a humic-rich ecological niche. Proc Natl Acad Sci U S A. 2012 Oct 23; 109(43). 17501-6. DOI:

10.1073/pnas.1206847109. Epub 2012 Oct 8.
Rama T., et al., 2000. Relationship between sporophore morphology and mushroom quality. Mushroom Science, XV (2): 725-731.
Rama T., et al., 1997. Review on mechanical properties and morphology related to mushroom quality. Plant Biomechanics: 295-300.
Romaine C.P. & Royse D.J., 2011. Biotechnology of mushroom (Agaricus bisporus) production. Encyclopaedia of Biotechnology in Agriculture and Food DOI: 10.1081/E-EBAF-120047309.
Savoie J.M., et al., 2013. Genetics and genomics of cultivated mushrooms, application to breeding of Agarics. Agricultural Applications, 2nd Edition. The Mycota XI. F. Kempken (Ed.). © Springer-Verlag Berlin Heidelberg 2013.
Sonnenberg A.S., et al., 2011. Breeding and strain protection in the button mushroom Agaricus bisporus. In: Savoie JM., Foulongne-Oriol M., Largeteau M., Barroso G. (eds). Proceedings of the 7th International Conference on Mushroom Biology and Mushrooms Products, vol. 1. Pp 7-15.
Steinbuch E., 1978. Factors affecting the quality and shrinkage losses of processed mushrooms. Mushroom Science X, 759-766.
Velcko A.J., et al., 2004. Expression of novel genes in Agaricus bisporus using an Agrobacterium-mediated transformation technique. Mushroom Science XVI, 591-597.
Xu J. et al., 1993. Localization of the mating type gene in Agaricus bisporus. Appl. Environ. Microbiol., 59, 9, 3044-3049.

## Claims

**1.** An *Agaricus bisporus* hybrid strain having the strain J10117 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4950, as first parent and an *Agaricus bisporus* white strain as a second parent, said hybrid strain being selected in the group consisting of:

- LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4945,
- LB906 having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4946, and

- Derivatives thereof wherein said derivatives are *Agaricus bisporus* hybrids comprising all the SSR molecular markers and/or P1N150 allelic sequences of the hybrid **LB811** having been deposited under the CNCM Accession Number I-4945 or of the hybrid **LB906** having been deposited under the CNCM Accession Number I-4946 or of the hybrid **J10117** having been deposited under the CNCM Accession Number I-4950,

wherein the P1N150 allelic combinations for LB811, LB906 and J10117 are the ones as in Table 17, and wherein the SSR molecular markers for LB811, LB906 and J10117 are the ones as in Table 15bis, wherein the said hybrid strain has an improved canning yield, the improved canning yield being defined as a higher canning yield, which is statistically significant at P < 0.05 or P < 0.01, compared to mushroom from a control commercial *Agaricus bisporus* strain A15.

**2.** The *Agaricus bisporus* hybrid strain of claim 1, wherein said derivatives are cells obtained from said LB811 or from said LB906 by somatic selection, tissue culture selection, single spore germination, multiple spore germination, matings between mycelium obtained from homokaryotic or heterokaryotic spores from these hybrids, selfing, repeated mating back to the initial culture, mutagenesis, trait conversion, introgressive trait conversion, inbreeding or transformation.

**3.** The *Agaricus bisporus* hybrid strain LB811, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number 1-4945.

**4.** The *Agaricus bisporus* hybrid strain LB906 having been deposited with the Collection Nationale de Cultures de Microorganismes (CNCM), Institut Pasteur, 25 rue du Docteur Roux, 75724 PARIS Cedex 15 on January 20, 2015, under the CNCM Accession Number I-4946.

**5.** A hybrid mushroom culture of *Agaricus bisporus* comprising the *Agaricus bisporus* hybrid of any one of claims 1-4.

**6.** A product incorporating the culture of claim 5, wherein said product is selected from the group consisting of: mycelium, spawn, inoculum, casing inoculum and colonised substrates including grain, compost and friable particulate matter.

**7.** Homokaryons obtained from the hybrid of any one of claims 1-4 or from the hybrid mushroom culture of claim 5.

**8.** A cell of the *Agaricus bisporus* culture of claim 5.

**9.** A mushroom produced by growing a crop of mushrooms from the culture of claim 5.

**10.** A method of producing hybrid mushrooms, said method comprising:

a) Inoculating a mushroom growth medium with a hybrid *Agaricus bisporus* mushroom hybrid of claim 1 to 4,
b) Maintaining said inoculated growth medium under conditions conductive to mushroom fruiting and
c) Collecting mushrooms from said growth medium.

**11.** A method of producing offspring of the hybrid mushroom culture of claim 5, comprising the steps of:

a) Obtaining spores from said hybrid mushroom culture,
b) Germinating said spores,
c) Transferring cultures from germinating spores to new growth media.

**12.** A process of producing a new hybrid mushroom culture, comprising mating the homokaryon of claim 8 or the hybrid of claims 1-4 with a second *Agaricus bisporus* homokaryon or heterokaryon.

**13.** The *Agaricus bisporus* hybrid strain J10117, having been deposited with the Collection Nationale de Cultures de Microorganismes (CNCM), Institut Pasteur, 25 rue du Docteur Roux, 75724 PARIS Cedex 15, on January 20, 2015, under the CNCM Accession Number I-4950.

**14.** Method of producing an *A. bisporus* strain producing mushrooms with improved canning yield comprising the steps of

i. providing propagating material of an hybrid *A. bisporus* strain according to the claim 1;

ii. growing said material and recovering a reproducing material therefrom;
iii. crossing the reproducing material obtained in ii) with the reproducing material of an *A. bisporus* strain which does not have the trait of increased canning yield,
iv. growing propagating material obtained from the cross and harvesting propagating material there from and
v. growing mushrooms from the propagating material harvested under iii) and selecting a mushrooms strain producing mushrooms with improved canning yield, wherein the improved canning yield being defined as a higher canning yield, which is statistically significant at $P < 0.05$ or $P < 0.01$, compared to mushroom from a control commercial *Agaricus bisporus* strain A15.

## Patentansprüche

1. *Agaricus bisporus* Hybridstamm, der den Stamm J10117, der am 20. Januar 2015 unter der CNCM-Hinterlegungsnummer I-4950 bei der Collection Nationale de Cultures de Microorganismes (CNCM), Institut Pasteur, 25 Rue du Docteur Roux, 75724 PARIS Cedex 15, hinterlegt wurde, als ersten Elter und einen weißen *Agaricus bisporus* Stamm als zweiten Elter umfasst, wobei der Hybridstamm ausgewählt ist aus der Gruppe, die besteht aus:

   - LB811, der am 20. Januar 2015 unter der CNCM-Hinterlegungsnummer I-4945 bei der Collection Nationale de Cultures de Microorganismes (CNCM), Institut Pasteur, 25 Rue du Docteur Roux, 75724 PARIS Cedex 15, hinterlegt wurde,
   - LB906, der am 20. Januar 2015 unter der CNCM-Hinterlegungsnummer I-4946 bei der Collection Nationale de Cultures de Microorganismes (CNCM), Institut Pasteur, 25 Rue du Docteur Roux, 75724 PARIS Cedex 15, hinterlegt wurde, und
   - Derivate davon, wobei die Derivate *Agaricus bisporus* Hybride sind, die sämtliche molekularen SSR-Marker und/oder P1N150-Allelsequenzen des unter der CNCM-Hinterlegungsnummer I-4945 hinterlegten Hybrids **LB811** oder des unter der CNCM-Hinterlegungsnummer I-4946 hinterlegten Hybrids **LB906** oder des unter der CNCM-Hinterlegungsnummer I-4950 hinterlegten Hybrids **J10117** umfassen,

   wobei die P1N150-Allelkombinationen für LB811, LB906 and J10117 die von Tabelle 17 sind,
   und wobei die molekularen SSR-Marker für LB811, LB906 und J10117 die von Tabelle 15b sind,
   wobei der Hybridstamm einen verbesserten Konservierungsertrag aufweist, wobei der verbesserte Konservierungsertrag als ein höherer Konservierungsertrag definiert ist, der bei $P < 0{,}05$ oder $P < 0{,}01$ im Vergleich zu Pilz von einem im Handel erhältlichen *Agaricus bisporus* Kontrollstamm A15 statistisch signifikant ist.

2. *Agaricus bisporus* Hybridstamm nach Anspruch 1, wobei die Derivate Zellen sind, die von dem LB811 oder von dem LB906 durch somatische Selektion, Gewebekulturselektion, Einzelsporenkeimung, Mehrsporenkeimung, Paarungen zwischen Myzel, das von homokaryotischen oder heterokaryotischen Sporen von diesen Hybriden erhalten wird, Selbstung, wiederholter Paarung zurück zur Ausgangskultur, Mutagenese, Merkmalsumwandlung, introgressive Merkmalsumwandlung, Inzucht oder Veränderung erhalten werden.

3. *Agaricus bisporus* Hybridstamm LB811, der am 20. Januar 2015 unter der CNCM-Hinterlegungsnummer I-4945 bei der Collection Nationale de Cultures de Microorganismes (CNCM), Institut Pasteur, 25 Rue du Docteur Roux, 75724 PARIS Cedex 15, hinterlegt wurde.

4. *Agaricus bisporus* Hybridstamm LB906, der am 20. Januar 2015 unter der CNCM-Hinterlegungsnummer I-4946 bei der Collection Nationale de Cultures de Microorganismes (CNCM), Institut Pasteur, 25 Rue du Docteur Roux, 75724 PARIS Cedex 15, hinterlegt wurde.

5. Hybridpilzkultur von *Agaricus bisporus,* der den *Agaricus bisporus* Hybriden nach einem der Ansprüche 1 bis 4 umfasst.

6. Produkt, das die Kultur nach Anspruch 5 aufnimmt, wobei das Produkt aus der Gruppe ausgewählt ist, die besteht aus: Myzel, Brut, Inokulum, Inokulum in Deckerde und kolonisierten Substraten, die Körner, Kompost und krümeliges Partikelmaterial umfassen.

7. Homokaryon, das von dem Hybriden nach einem der Ansprüche 1 bis 4 oder von der Hybridpilzkultur nach Anspruch 5 erhalten wird.

8. Zelle der *Agaricus bisporus* Kultur nach Anspruch 5.

9. Pilz, der durch Züchten einer Ernte von Pilzen aus der Kultur nach Anspruch 5 hergestellt wird.

10. Verfahren zur Herstellung von Hybridpilzen, wobei das Verfahren Folgendes umfasst:

a) Inokulieren eines Pilznährmediums mit einem hybriden *Agaricus bisporus* Pilzhybriden nach Anspruch 1 bis 4,
b) Halten des inokulierten Nährmediums unter Bedingungen, die zu Pilzfruchtbildung führen, und
c) Sammeln Pilzen von dem Nährmedium.

11. Verfahren zur Herstellung von Brut der Hybridpilzkultur nach Anspruch 5, das die folgenden Schritte umfasst:

a) Erhalten von Sporen von der Hybridpilzkultur,
b) Keimen der Sporen,
c) Überführen von Kulturen von keimenden Sporen in das neue Nährmedium.

12. Verfahren zur Herstellung einer neuen Hybridpilzkultur, das das Paaren des Homokaryons nach Anspruch 8 oder des Hybriden nach Anspruch 1 bis 4 mit einem zweiten *Agaricus bisporus* Homokaryon oder Heterokaryon umfasst.

13. *Agaricus bisporus* Hybridstamm J10117, der am 20. Januar 2015 unter der CNCM-Hinterlegungsnummer I-4950 bei der Collection Nationale de Cultures de Microorganismes (CNCM), Institut Pasteur, 25 Rue du Docteur Roux, 75724 PARIS Cedex 15, hinterlegt wurde.

14. Verfahren zur Herstellung eines A. *bisporus* Stamms, das Pilze mit verbessertem Konservierungsertrag herstellt und die folgenden Schritte umfasst:

i. Bereitstellen von Ausbreitungsmaterial eines A. *bisporus* Hybridstamms nach Anspruch 1;
ii. Züchten des Materials und Rückgewinnen eines Fortpflanzungsmaterials davon;
iii. Kreuzen des in ii) erhaltenen Fortpflanzungsmaterials mit dem Fortpflanzungsmaterial eines A. *bisporus* Stamms, der nicht die Eigenschaft des erhöhten Konservierungsertrags aufweist,
iv. Züchten von Ausbreitungsmaterial, das von der Kreuzung erhalten wurde, und Ernten des Ausbreitungsmaterials davon und
v. Züchten von Pilzen aus dem in iii) geernteten Ausbreitungsmaterial und Auswählen eines Pilzstamms, der Pilze mit verbessertem Konservierungsertrag herstellt, wobei der verbesserte Konservierungsertrag als ein höherer Konservierungsertrag definiert ist, der bei $P < 0,05$ oder $P < 0,01$ im Vergleich zu Pilz von einem im Handel erhältlichen *Agaricus bisporus* Kontrollstamm A15 statistisch signifikant ist.

## Revendications

1. Souche hybride *d'Agaricus bisporus* ayant la souche J10117 déposée à la Collection Nationale de Cultures de Microorganismes (CNCM), Institut Pasteur, 25 rue du Docteur Roux, 75724 PARIS Cedex 15, le 20 janvier 2015, sous le numéro d'accès CNCM I-4950, en tant que premier parent, et une souche *d'Agaricus bisporus* blanc en tant que deuxième parent, ladite souche hybride étant choisie dans le groupe constitué par :

- LB811, déposée à la Collection Nationale de Cultures de Microorganismes (CNCM), Institut Pasteur, 25 rue du Docteur Roux, 75724 PARIS Cedex 15, le 20 janvier 2015, sous le numéro d'accès CNCM I-4945,
- LB906, déposée à la Collection Nationale de Cultures de Microorganismes (CNCM), Institut Pasteur, 25 rue du Docteur Roux, 75724 PARIS Cedex 15, le 20 janvier 2015, sous le numéro d'accès CNCM I-4946, et
- leurs dérivés, lesquels dérivés sont des hybrides *d'Agaricus bisporus* comprenant tous les marqueurs moléculaires SSR et/ou séquences alléliques P1N150 de l'hybride **LB811** déposé sous le numéro d'accès CNCM I-4945 ou de l'hybride **LB906** déposé sous le numéro d'accès CNCM I-4946 ou de l'hybride **J10117** déposé sous le numéro d'accès CNCM I-4950,

dans laquelle les combinaisons alléliques P1N150 pour LB811, LB906 et J10117 sont celles présentées dans le tableau 17,
et dans laquelle les marqueurs moléculaires SSR pour LB811, LB906 et J10117 sont ceux présentés dans le tableau 15bis,

laquelle souche hybride a un rendement de mise en conserve amélioré, le rendement de mise en conserve étant défini par un rendement de mise en conserve plus élevé, qui est statistiquement significatif à P < 0,05 ou P < 0,01, que celui d'un champignon témoin provenant d'une souche d'*Agaricus bisporus* A15 du commerce.

**2.** Souche hybride d'*Agaricus bisporus* selon la revendication 1, dans laquelle lesdits dérivés sont des cellules obtenues à partir de ladite LB811 ou à partir de ladite LB906 par sélection somatique, sélection en culture tissulaire, germination de spores isolés, germination de spores multiples, croisements de mycéliums obtenus à partir de spores homocaryotes ou hétérocaryotes provenant de ces hybrides, autofécondation, croisements répétés ramenant à la culture initiale, mutagenèse, conversion de traits, conversion de traits introgressive, endogamie, ou transformation.

**3.** Souche hybride d'*Agaricus bisporus* LB811, déposée à la Collection Nationale de Cultures de Microorganismes (CNCM), Institut Pasteur, 25 rue du Docteur Roux, 75724 PARIS Cedex 15, le 20 janvier 2015, sous le numéro d'accès CNCM I-4945.

**4.** Souche hybride d'*Agaricus bisporus* LB906, déposée à la Collection Nationale de Cultures de Microorganismes (CNCM), Institut Pasteur, 25 rue du Docteur Roux, 75724 PARIS Cedex 15 le 20 janvier 2015, sous le numéro d'accès I-4946.

**5.** Culture de champignon hybride d'*Agaricus bisporus* comprenant l'hybride d'*Agaricus bisporus* de l'une quelconque des revendications 1 à 4.

**6.** Produit incorporant la culture de la revendication 5, lequel produit est choisi dans le groupe constitué par : un mycélium, une progéniture, un inoculum, un inoculum d'entretien de croissance, et les substrats colonisés y compris les grains, le compost et les matières particulaires friables.

**7.** Homocaryons obtenus à partir de l'hybride de l'une quelconque des revendications 1 à 4 ou de la culture de champignon hybride de la revendication 5.

**8.** Cellule de la culture *d'Agaricus bisporus* de la revendication 5.

**9.** Champignon produit par croissance d'une récolte de champignons provenant de la culture de la revendication 5.

**10.** Procédé de production de champignons hybrides, ledit procédé comprenant :

a) l'inoculation d'un milieu de croissance de champignon avec un hybride de champignon *Agaricus bisporus* des revendications 1 à 4,
b) le maintien dudit milieu de culture inoculé dans des conditions propices à la fructification des champignons, et
c) la collecte des champignons à partir dudit milieu de croissance.

**11.** Procédé de production d'une descendance de la culture de champignon hybride de la revendication 5, comprenant les étapes suivantes :

a) obtention de spores à partir de ladite culture de champignon hybride,
b) germination desdits spores,
c) transfert des cultures issues des spores en germination sur un nouveau milieu de croissance.

**12.** Procédé de production d'une nouvelle culture de champignon hybride, comprenant le croisement de l'homocaryon de la revendication 8 ou de l'hybride des revendications 1 à 4 avec un deuxième homocaryon ou hétérocaryon d'*Agaricus bisporus.*

**13.** Souche hybride d'*Agaricus bisporus* J10117, déposée à la Collection Nationale de Cultures de Microorganismes (CNCM), Institut Pasteur, 25 rue du Docteur Roux, 75724 PARIS Cedex 15, le 20 janvier 2015, sous le numéro d'accès CNCM I-4950.

**14.** Procédé de production d'une souche d'A. *bisporus* produisant des champignons avec un rendement de mise en conserve amélioré, comprenant les étapes suivantes :

i. obtention d'un matériau de propagation d'une souche hybride d'A. *bisporus* selon la revendication 1 ;
ii. croissance dudit matériau et récupération d'un matériau reproducteur à partir de celui-ci ;
iii. croisement du matériau reproducteur obtenu en ii) avec le matériau reproducteur d'une souche d'A. *bisporus* qui n'a pas le trait de rendement plus élevé de mise en conserve,
iv. croissance du matériau de propagation obtenu à partir du croisement et récolte du matériau de propagation à partir de celui-ci et
v. croissance de champignons à partir du matériau de propagation récolté en iii) et sélection d'une souche de champignons produisant des champignons ayant un rendement de mise en conserve amélioré, lequel rendement de mise en conserve amélioré est défini par un rendement de mise en conserve plus élevé, qui est statistiquement significatif à $P < 0{,}05$ ou $P < 0{,}01$, que celui d'un champignon témoin provenant d'une souche *d'Agaricus bisporus* A15 du commerce.

**Figure 1**

A

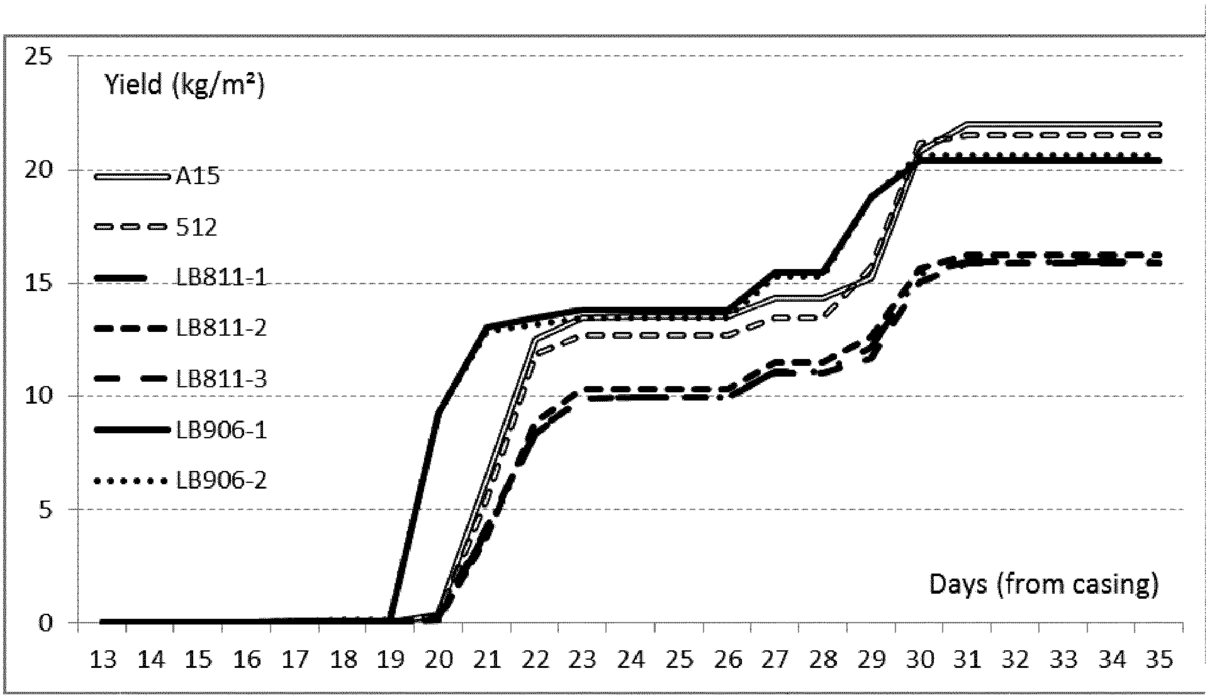

Yield (kg/m²)
A15
512
LB811-1
LB811-2
LB811-3
LB906-1
LB906-2
Days (from casing)
0
5
10
15
20
25
13 14 15 16 17 18 19 20 21 22 23 24 25 26 27 28 29 30 31 32 33 34 35


B

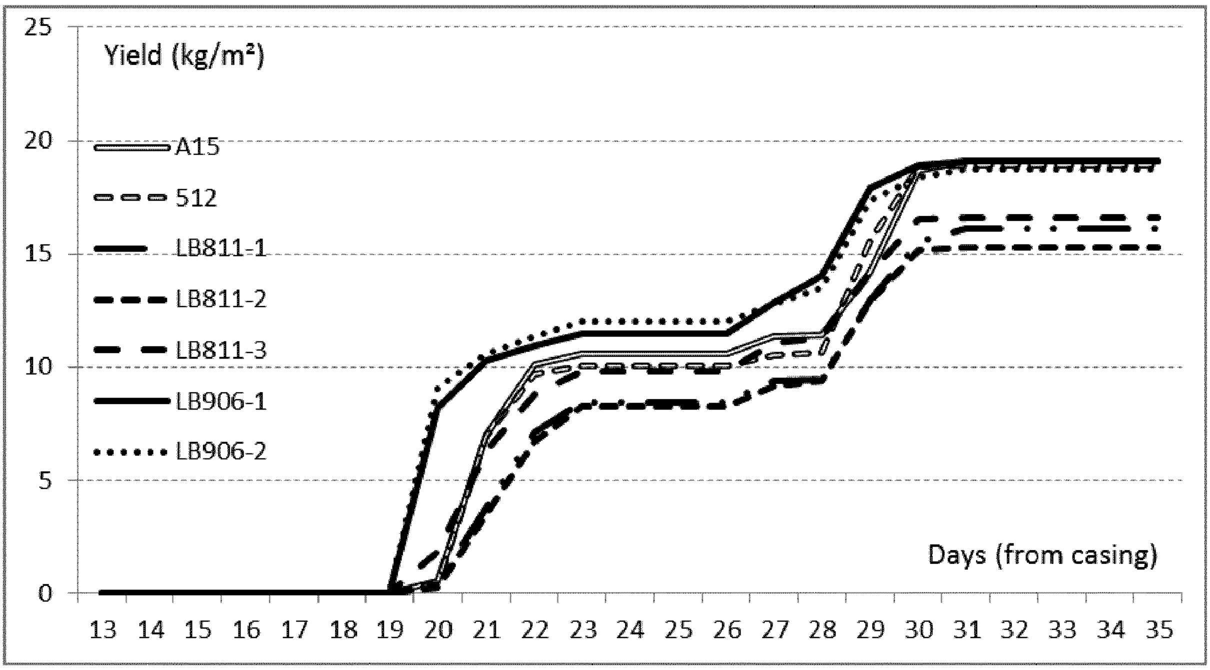

Yield (kg/m²)
A15
512
LB811-1
LB811-2
LB811-3
LB906-1
LB906-2
Days (from casing)
0
5
10
15
20
25
13 14 15 16 17 18 19 20 21 22 23 24 25 26 27 28 29 30 31 32 33 34 35

Figure 2

A

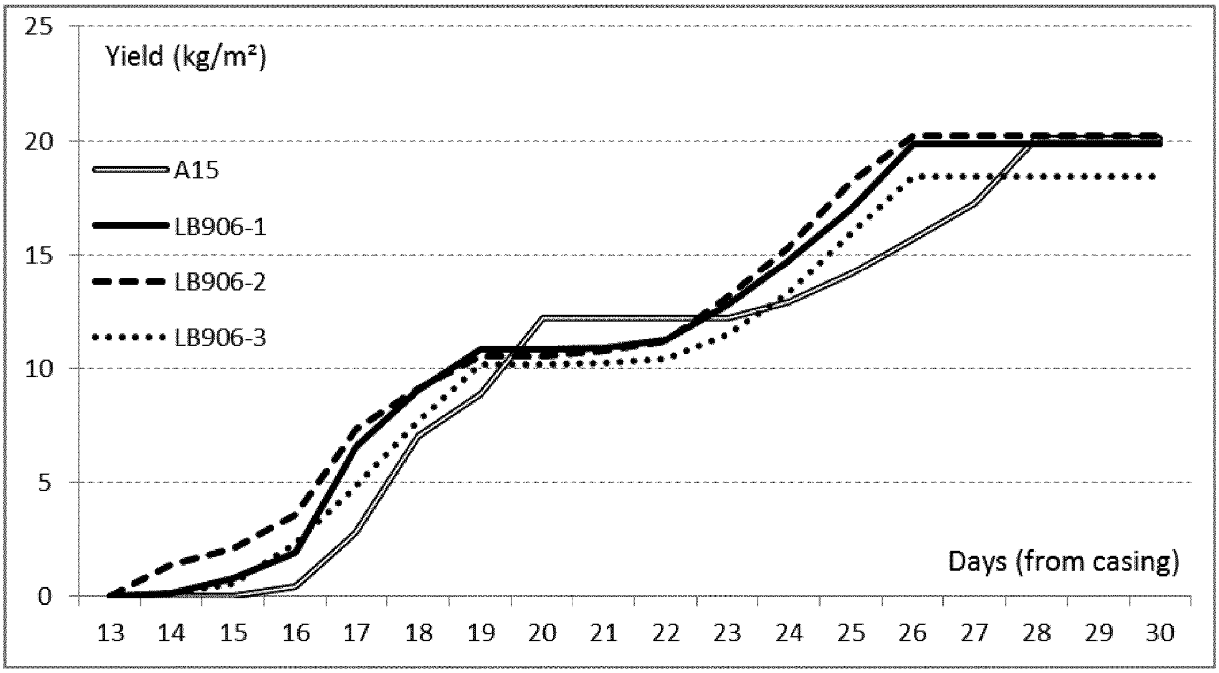
Yield (kg/m²)
A15
LB906-1
LB906-2
LB906-3
Days (from casing)
25
20
15
10
5
0
13 14 15 16 17 18 19 20 21 22 23 24 25 26 27 28 29 30

B

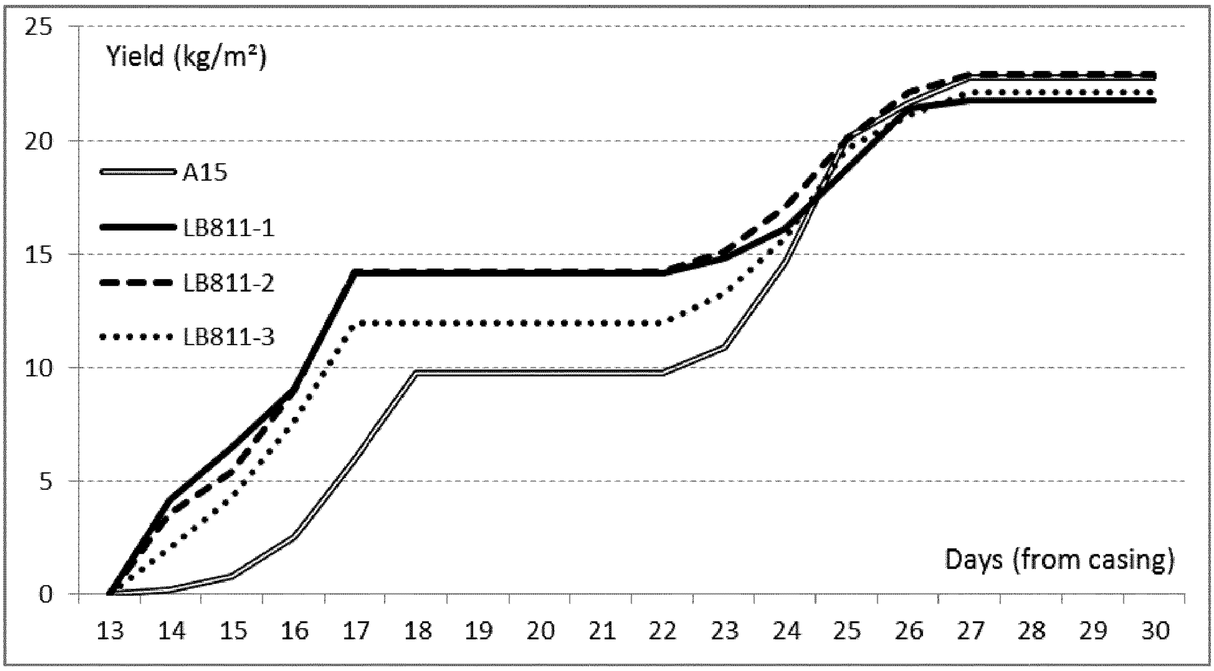
Yield (kg/m²)
A15
LB811-1
LB811-2
LB811-3
Days (from casing)
25
20
15
10
5
0
13 14 15 16 17 18 19 20 21 22 23 24 25 26 27 28 29 30

Figure 3

A

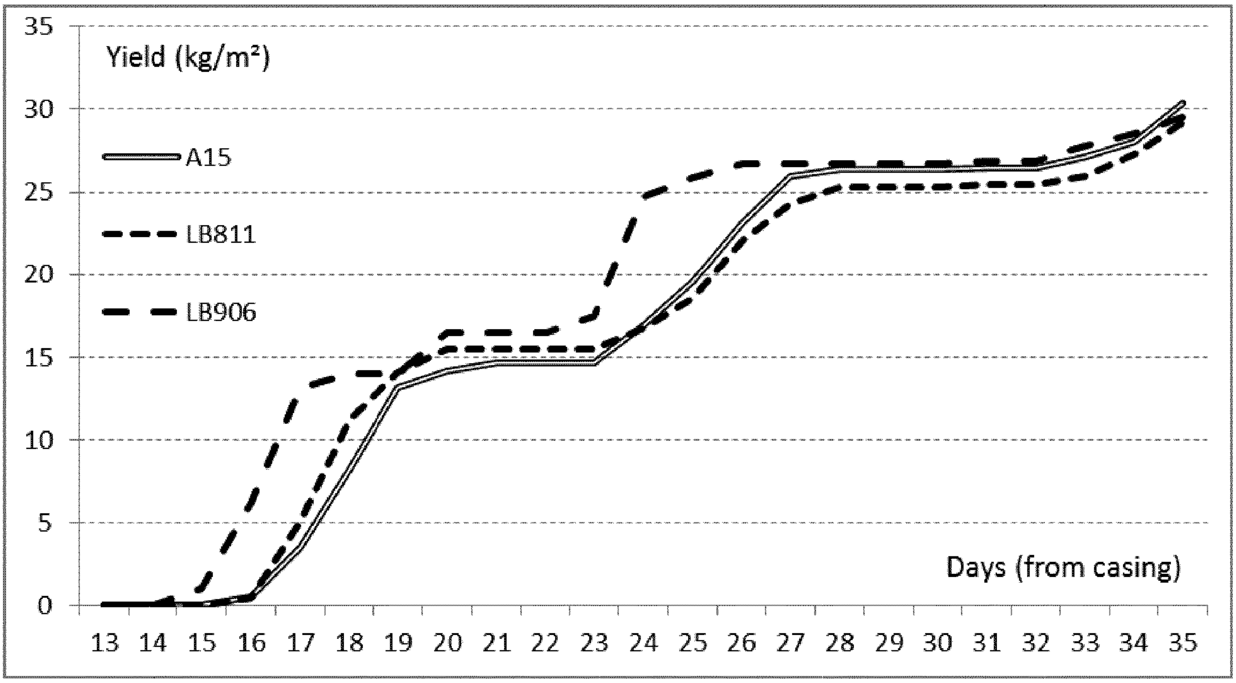
35
30
25
20
15
10
5
0
Yield (kg/m²)
A15
LB811
LB906
Days (from casing)
13 14 15 16 17 18 19 20 21 22 23 24 25 26 27 28 29 30 31 32 33 34 35

B

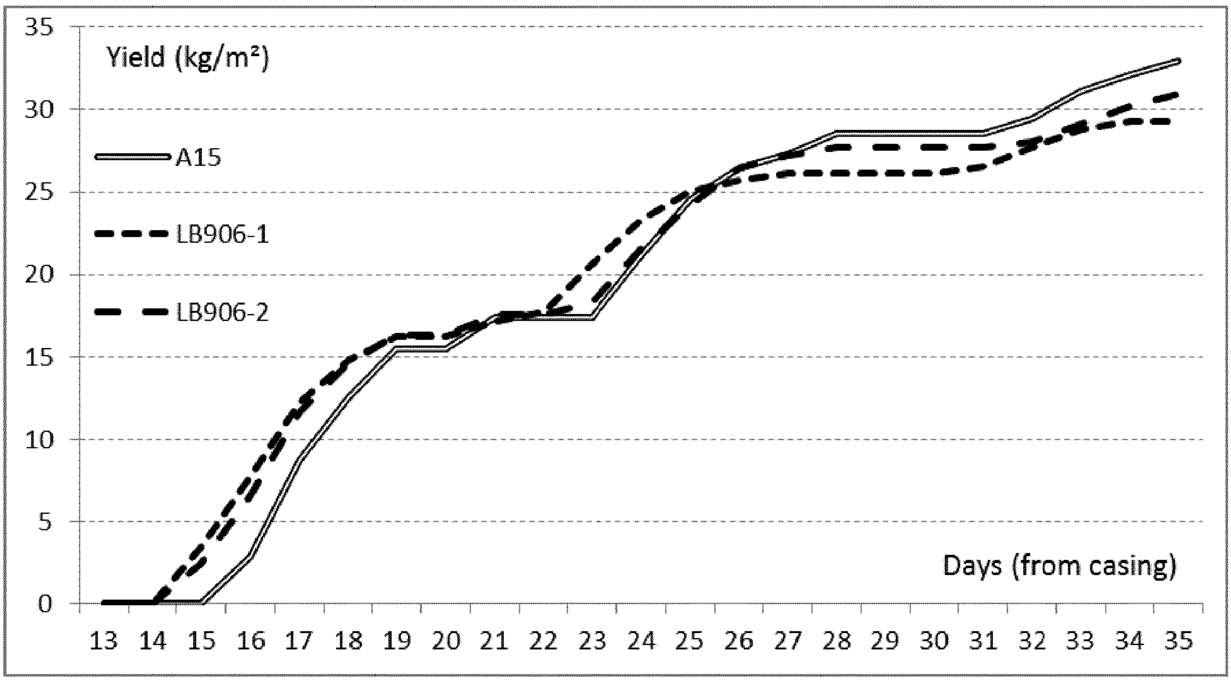
35
30
25
20
15
10
5
0
Yield (kg/m²)
A15
LB906-1
LB906-2
Days (from casing)
13 14 15 16 17 18 19 20 21 22 23 24 25 26 27 28 29 30 31 32 33 34 35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5832659 A
- US 8663969 B
- US 20100218294 A
- US 2100154079 A
- WO 2015114612 A
- US 20080182321 A
- US 2010154079 A
- EP 15305073

### Non-patent literature cited in the description


- **BEELMAN R.B. et al.** Bioactive components in Agaricus bisporus of nutritional, medicinal or biological importance. *Mushroom Science,* 2008, vol. XVII, 1-16
- **CALLAC P. et al.** Morphological, genetic, and interfertility analyses reveal a novel, tetrasporic variety of Agaricus bisporus from the Sonoran Desert of California. *Mycologia,* 1993, vol. 85, 835-851
- **CALLAC P. et al.** A novel homothallic variety of Agaricus bisporus comprises rare tetrasporic isolates from Europe. *Mycologia,* 2003, vol. 95, 222-231
- **CHEN S ; KANAYA N.** Protective effects of Agaricus bisporus against cancer and metabolic diseases. *Mushroom Science,* 2012, vol. XVIII, 28-33
- **COALE C.W.J. ; BUTZ W.T.** Impact of selected economic variables on the profitability of commercial mushroom processing operations. *Mushroom Science,* 1972, vol. VIII, 231-237
- **DONKER H.C.W. et al.** NMR imaging of white button mushrooms (Agaricus bisporus) at various magnetic fields. *Magnetic Resonance Imaging,* 1996, vol. 14 (10), 1205-1215
- Mushroom pests and disease control. **FLETCHER J.T. ; GAZE R.H.** A colour handbook. Manson Publishing, 2008, 7-17, 18-21, 109-123
- Agaricus bisporus cultivars: Hidden diversity beyond apparent uniformity?. **FOULONGNE-ORIOL M. et al.** Proceedings of the Seventh International Conference on Mushroom Biology and Mushroom Products. 2011, vol. 2, 9-16
- **FOULONGNE-ORIOL M. et al.** Quantitative trait locus mapping of yield-related components and oligogenic control of the cap color of the button mushroom, Agaricus bisporus. *Applied and Environmental Microbiology,* 2012, vol. 78 (7), 2422-2434
- **FOULONGNE-ORIOL M. et al.** Novel microsatellite markers suitable for genetic studies in the white button mushroom Agaricus bisporus. *Appl. Microbiol. Biotechnol.,* 2009
- **FOULONGNE-ORIOL M. et al.** Development of polymorphic microsatellite markers issued from pyrosequencing technology for the medicinal mushroom Agaricus subrufescens. *FEMS Microbiol. Lett.,* 2012, vol. 334, 119-126
- **FRITSCHE G.** Breeding Agaricus bisporus at the Mushroom Experimental Station, Horst. *The Mushroom Journal,* 1983, vol. 122, 49-53
- **GAO W. et al.** Genetic variation and combining ability analysis of bruising sensitivity in Agaricus bisporus. *PloS one,* 2013, vol. 8 (10), e76826
- **IMBERNON M. et al.** Allelic polymorphism at the mating-type locus in Agaricus bisporus var. burnettii, and confirmation of the dominance of its tetrasporic trait. *Mushroom Science,* 1995, vol. XIV (1), 11-19
- **KERRIGAN R.W.K. et al.** The heterothallic life cycle of Agaricus bisporus var. burnettii, and the inheritance of its tetrasporic trait. *Exp Mycol,* 1994, vol. 18, 193-210
- **KERRIGAN R.W.K. et al.** Meiotic behavior and linkage relationships in the secondary homothallic fungus Agaricus bisporus. *Genetics,* 1993, vol. 133, 225-236
- **MORIN E. et al.** Genome sequence of the button mushroom Agaricus bisporus reveals mechanisms governing adaptation to a humic-rich ecological niche. *Proc Natl Acad Sci U S A.,* 08 October 2012, vol. 109 (43), 17501-6
- **RAMA T. et al.** Relationship between sporophore morphology and mushroom quality. *Mushroom Science,* 2000, vol. XV (2), 725-731
- **RAMA T. et al.** Review on mechanical properties and morphology related to mushroom quality. *Plant Biomechanics,* 1997, 295-300
- **ROMAINE C.P. ; ROYSE D.J.** Biotechnology of mushroom (Agaricus bisporus) production. *Encyclopaedia of Biotechnology in Agriculture and Food,* 2011

- Genetics and genomics of cultivated mushrooms, application to breeding of Agarics. **SAVOIE J.M. et al.** Agricultural Applications. Springer-Verlag, 2013
- Breeding and strain protection in the button mushroom Agaricus bisporus. **SONNENBERG A.S. et al.** Proceedings of the 7th International Conference on Mushroom Biology and Mushrooms Products. 2011, vol. 1, 7-15
- **STEINBUCH E.** Factors affecting the quality and shrinkage losses of processed mushrooms. *Mushroom Science,* 1978, vol. X, 759-766
- **VELCKO A.J. et al.** Expression of novel genes in Agaricus bisporus using an Agrobacterium-mediated transformation technique. *Mushroom Science,* 2004, vol. XVI, 591-597
- **XU J. et al.** Localization of the mating type gene in Agaricus bisporus. *Appl. Environ. Microbiol.,* 1993, vol. 59 (9), 3044-3049